# EUROPÄISCHE PATENTSCHRIFT

(11) **EP 4 052 033 B1**
(45) Veröffentlichungstag und Bekanntmachung des Hinweises auf die Patenterteilung: **29.01.2025**
(21) Anmeldenummer: 20797746.3
(22) Anmeldetag: 27.10.2020
(51) Int. Cl.: G01N 33/487

(54) **ELEKTROPHYSIOLOGISCHES MESSGERÄT UND MESSVERFAHREN ZUR ERFASSUNG MINDESTENS EINES ELEKTRISCHEN MESSWERTS AN EINER BIOLOGISCHEN ZELLPROBE**
ELECTROPHYSIOLOGICAL MEASURING APPARATUS AND MEASUREMENT METHOD FOR DETECTING AT LEAST ONE ELECTRICAL MEASURED VALUE OF A BIOLOGICAL CELL SAMPLE
APPAREIL DE MESURE ÉLECTROPHYSIOLOGIQUE ET PROCÉDÉ DE MESURE POUR ACQUÉRIR AU MOINS UNE VALEUR DE MESURE ÉLECTRIQUE AU NIVEAU D'UN ÉCHANTILLON DE CELLULES BIOLOGIQUES

(30) Priorität: 28.10.2019 DE 102019129042
(43) Veröffentlichungstag der Anmeldung: 07.09.2022
(73) Patentinhaber: ChanPharm GmbH, 1190 Wien (AT)
(72) Erfinder: HERING, Steffen, 1190 Wien (AT)
(74) Vertreter: v. Bezold & Partner Patentanwälte - PartG mbB
(86) Internationale Anmeldenummer: PCT/EP2020/080163
(87) Internationale Veröffentlichungsnummer: WO 2021/083885

(56) Entgegenhaltungen:
- DE-A1- 10 254 158
- US-A1- 2004 182 707
- US-A1- 2006 129 043
- US-A1- 2010 304 425
- US-A1- 2014 348 706

## Beschreibung

Die Erfindung betrifft elektrophysiologische Messgeräte und elektrophysiologische Messverfahren zur Erfassung mindestens eines elektrischen Messwerts an einer biologischen Zellprobe, insbesondere ein Messgerät und ein Messverfahren zur Erfassung von einem Aktionspotential an mindestens einer Zellmembran der Zellprobe und/oder einem lonenstrom durch mindestens eine Zellmembran der Zellprobe. Es werden auch Substrate zur Aufnahme biologischer Zellen und deren Anwendungen beschrieben. Die Erfindung ist bei der Handhabung und insbesondere Charakterisierung von biologischen Zellproben, insbesondere von einzelnen Zellen oder Zellaggregaten, in der Pharmakologie, Medizin, Biochemie und/oder Biomedizin anwendbar.

In der vorliegenden Beschreibung wird auf den folgenden Stand der Technik Bezug genommen, der den technischen Hintergrund der Erfindung darstellt:
[1] EP 1 040 349 B1;
[2] O. P. Hamill et al. in "Pflugers Arch." 1981 Aug; 391(2):85-100;
[3] EP 1 349 916 A2;
[4] J. Abbott et al. in "Acc. Chem. Res." 51 (2019) 600-608;
[5] A. Cerea et al. in "Lab Chip" 18 (2018) 3492-3500;
[6] X. Xie et al. in "ACS Nano" 9 (2015) 11667-11677;
[7] X. Xie et al. in "ACS Nano" 7 (2013) 4351-4358;
[8] K. Li et al. in "Comprehensive Biotechnology" Kapitel 5.11, S.125-139 (Elsevier B. V. 2011, DOI: 10.1016/B978-0-08-088504-9.00497-9);
[9] M. R. Angle et al. in "Biophysical Journal" 107 (2014) 2091-2100;
[10] "Nanostrukturierte Metallelektroden zur funktionalen Kopplung an neuronale Zellen", Dorothea Brüggemann, Dissertation, RWTH Aachen, Schriften des Forschungszentrums Jülich, Band 9, ISBN 978-3-89336-627-9;
[11] WO 2010/031506 A1;
[12] A. M. Shum et al. in "Adv. Mater." 29 (2017) 1602448;
[13] EP 1 953 812 A1;
[14] C. Morez et al. in "Biomaterials" 70, 94-104;
[15] M. A. Bucaro et al. in " ACSNANO" 6 (2012), 6222-6230;
[16] DE 10 2017 130 518;
[17] R. Wang et al. in "Scientific reports" 8 (2018) Artikel-Nr.: 14205;
[18] "Entwicklung eines autokontraktilen Herzmuskelmodells zur funktionalen Medikamenten-und Toxinforschung" Matthias Goßmann, Dissertation, Universität Duisburg-Essen, 2015, (https://duepublico2.uni-due.de/servlets/MCRFileNodeServlet/duepublico_ derivate_00040212/DissGossmann.pdf;
[19] M. Goßmann et al. in "Cell. Physiol. Biochem." 2016; 38:1182 - 1198;
[20] E. Musk et al. "An integrated brain-machine interface platform with thousands of channels" (http://dx.doi.org/10.1101/703801);
[21] DE 198 27 957 A1;
[22] US 2006/0 163 062 A1;
[23] Y. I. Zilberter et al. in "Pflugers Arch." 1982 Aug, 394(2):150-5; und
[24] US 2006/129043 A1.

Elektrophysiologische Untersuchungen an Zellproben aus mindestens einer biologischen Zelle oder Zellaggregaten umfassen insbesondere die Messung von lonenströmen durch die Zellmembran ("patch-clamp"-Messung) oder die Messung von Aktionspotentialen an der Zellmembran ("current-clamp"-Messung). Bei dem älteren "voltage-clamp"-Verfahren wird ein Kompensationsstrom in die Probe geleitet, um die Membranspannung konstant zu halten, wohingegen bei "patch-clamp"-Messungen wegen der geringen Stromamplituden auf die Injektion eines Kompensationsstromes während der Messung verzichtet werden kann. Es ist bekannt, elektrophysiologische Messungen an Pipettenspitzen oder mit planaren Messgeräten durchzuführen, bei denen eine einzelne Zelle oder ein rekonstituierte Membransystem auf einer Pore in einer ebenen Oberfläche ("patch-Pore") positioniert wird ([1], [2]). Es können mehrere Poren in einer Oberfläche vorhanden sein und somit mehrere Einzelzellen gleichzeitig mit der "patch-clamp" Technik ([2]) untersucht werden (Parallelisierung der Untersuchung). Die (Multiarray-)-"patch-clamp"-Methode bzw. "patch-clamp"-Chips werden z. B. in [1] und [3] beschrieben. Die Zellmembran einer einzelnen Zelle oder auch die Membranen mehrerer Zellen bilden bei den patch-clamp-Messungen eine feste Verbindung mit dem Rand einer Saugpore (auch als patch-Pore bezeichnet), die mit einer Saugvorrichtung verbunden ist. Am Rand der Saugpore wird der so genannte Sealwiderstand gebildet. Bei einer Variante der patch-clamp-Messungen, der so genannten "cell attached"-Messung" wird die Membran in der Pore nicht zerstört, sondern bleibt intakt (siehe [23]). Ein Nachteil dieser Messungen ist, dass das Membranpotential (im Gegensatz zur so genannten "whole cell"-Messung) nicht erfasst werden kann.

Zur weiteren Parallelisierung von elektrophysiologischen Untersuchungen ist die Verwendung von Nanoelektroden-Arrays vorgeschlagen worden (siehe zum Beispiel [4] bis [8]). Ein Nanoelektroden-Array umfasst eine Vielzahl von kompakten ([4], [8]) oder hohlen ([5] bis [8]) Elektrodenspitzen mit typischen Durchmessern im sub-Mikrometer-Bereich, die zum Beispiel als Matrixanordnung von einem festen Substrat abstehen. Um eine intrazelluläre Messung (Messung im Zellinneren) durchzuführen, d.h. um einen direkten elektrischen Kontakt zwischen der Elektrode und dem Zytosol im Zellinneren herzustellen, müssen die Elektrodenspitzen die Zellmembran durchdringen (penetrieren). Andernfalls würde ohne ein Eindringen der Elektrodenspitzen in das Zellinnere das Zytosol von der Elektrodenspitze durch die Zellmembran isoliert werden, so dass praktisch nur eine extrazelluläre Messung (Messung an der Zellaußenseite) durchgeführt werden könnte. Die extrazelluläre Messung ist jedoch weniger aussagekräftig und wegen der relativ geringen Potentiale ungenauer als die intrazelluläre Messung.

Die Penetration der Zellmembran kann spontan erfolgen oder durch eine Elektroporation induziert werden [4]. Die spontane Penetration hat die Nachteile, dass es sich um ein seltenes, nicht reproduzierbar realisiertes Ereignis handelt und die Elektrode oft nur unzureichend in die Zelle eindringt. Die Elektroporation hingegen kann mit einer unerwünschten Beeinflussung der Zelle und damit des Messergebnisses der elektrophysiologischen Untersuchung verbunden sein. Hohle Nanoelektroden der Nanoelektroden-Arrays aus [5] bis [8] erlauben neben einer elektrophysiologischen Messung oder sensorischen Aufgaben auch die Zufuhr von Biomolekülen in das Zellinnere. Neben den genannten Nachteilen hinsichtlich des Eindringens der Elektroden in die Zellen haben die hohlen Nanoelektroden weitere Nachteile aufgrund der beschränkten Miniaturisierbarkeit und Komplexität des Substrataufbaus.

Die Messung der Kraft, die zum Durchdringen der Zellmembran mit einer Nanoelektrode erforderlich ist, mittels Techniken der Atomkraftmikroskopie (AFM) wird in [9] beschrieben. Die untersuchte Elektrode wird auf einem biegsamen AFM-Träger angeordnet und mit diesem in eine Zelle eingeführt. Aus dieser Technik ergeben sich zwar Hinweise auf die Kräfte, die für die Penetration erforderlich sind. Für praktische, routinemäßige elektrophysiologische Untersuchungen wäre diese Technik jedoch ungeeignet, da sie auf die Anwendung der AFM-Technik und auf Messungen mit einzelnen Elektroden beschränkt ist.

Weitere Anwendungen von Nanoelektroden-Arrays sind in [10] und [11] beschrieben. Gemäß [10] werden mit nanostrukturierten Metallelektroden extrazelluläre Signale von biologischen Zellen abgeleitet. Die nanostrukturierten Metallelektroden sind für intrazelluläre Messungen ungeeignet. In [11] wird vorgeschlagen, elektrophysiologische Potentiale zur Erzeugung elektrischer Ströme auszunutzen. Um ein Eindringen der Nanoelektroden in die Zellen zu fördern, sind die Spitzen der Nanoelektroden mit einer Beschichtung versehen, die eine Endozytose der Membran aktiviert und damit die Aufnahme der Elektroden in den Innenraum der Zellen erleichtert. Diese Technik ist für elektrophysiologische Untersuchungen ungeeignet, da die Endozytose-Beschichtung die Zellen in unerwünschter Weise beeinflussen kann.

Ein weiterer Nachteil herkömmlicher Nanoelektroden-Arrays kann sich durch eine natürliche Zellbewegung ergeben, die adhärente biologische Zellen aufgrund einer laufenden Umordnung ihrer Zell-Substrat-Kontakte ausführen können. Zellen können während der Messung über ein Substrat mit Nanoelektroden wandern, wodurch das Messergebnis verfälscht wird. Die natürliche Zellbewegung kann durch eine Adhäsions-mindernde Substratbeschichtung eingeschränkt werden. Die Substratbeschichtung hat jedoch den Nachteil, dass sie die Physiologie der Zellen beeinflussen kann.

Das Interesse an einer Parallelisierung der Untersuchung von Zellen besteht nicht nur, um die Zahl der gleichzeitigen Messungen zu erhöhen. Vielmehr können bei der Anordnung einer Vielzahl biologischer Zellen auf einem Substrat mit einer strukturierten Oberfläche physiologische Bedingungen, wie zum Beispiel in einem Gewebe, nachgebildet werden. Strukturierte Substrate zur Aufnahme einer Vielzahl biologischer Zellen, wie sie zum Beispiel in [12] beschrieben sind, erlauben die Beobachtung von biochemisch induzierten Zellveränderungen und deren Übertragung von Zelle zu Zelle. Auch in diesem Fall sollen Zellbewegungen möglichst unterbunden werden, was bei den aus [12] bekannten Substraten nur ungenügend möglich ist. In [13] sind deformierbare Substrate beschrieben, die biologische Zellen aufnehmen können, aber nur für mechanische Verschiebungen biologischer Zellenproben geeignet wären. Aus [14] und [15] sind strukturierte Substrate mit so genannten Microgrooves bekannt, welche die Kultivierung adhärenter Zellen beeinflussen sollen, in der Praxis aber eine beschränkte Wirksamkeit haben können. Generell kann auch von Nachteil sein, wenn herkömmliche Substrate Beschichtungen, wie z.B. Matrigel oder Fibronektin, tragen, die an Zellen unerwünschte biochemische Veränderungen hervorrufen können.

Weitere elektrophysiologische Untersuchungen an biologischen Zellen werden in [21] und [22] beschrieben, wobei die Zellen mit einem Unterdruck an Elektroden mit typischen Durchmessern im mm- oder µm-Bereich gesaugt und gehaltert werden. In [21] wird die Verwendung einer Mikroelektrode mit einem Durchmesser oberhalb 10 µm vorgeschlagen, die mittig im Inneren eines zylindrischen Elektrodenmantels sitzt, in dem ein Unterdruck erzeugt wird und der eine Saugeinrichtung bildet. Gemäß [22] ist die Elektrode zentral in einer Saugöffnung und vor diese vorragend angeordnet. Diese Techniken können aufgrund der relativ großen Elektrodendurchmesser Nachteile sowohl in Bezug auf den Platzbedarf der Elektroden als auch hinsichtlich der elektrophysiologischen Signalableitung haben. Die Technik gemäß [21] hat beschränkte Anwendungen, da insbesondere cell attached-Messungen nicht möglich sind.

Aus [24] ist ein elektrophysiologisches Messgerät bekannt, bei dem eine Zelle unter der Wirkung eines Saugdrucks auf einer Pore gehalten wird, in der eine spitzenförmige Elektrode angeordnet ist. Maßnahmen für eine stabile Halterung der Zelle auf der Pore sind in [24] nicht offenbart.

Die Aufgabe der Erfindung ist es, ein verbessertes elektrophysiologisches Messgerät und/oder ein verbessertes elektrophysiologisches Messverfahren zur Erfassung mindestens eines elektrischen Messwerts an einer biologischen Zellprobe bereitzustellen, mit denen Nachteile und Beschränkungen herkömmlicher Techniken vermieden werden können. Die Erfindung soll insbesondere elektrophysiologische Untersuchungen mit erhöhter Genauigkeit, Reproduzierbarkeit und Zuverlässigkeit ermöglichen, für die intrazelluläre Messung an Zellen geeignet sein, routinemäßige Anwendungen erlauben und/oder einen erweiterten Anwendungsbereich haben. Gemäß einem weiteren Gesichtspunkt soll die Erfindung ein verbessertes elektrophysiologisches Messgerät und/ oder ein verbessertes elektrophysiologisches Messverfahren bereitstellen, wobei eine bessere Haftung von Zellen auf Substratoberflächen ermöglicht wird.

Diese Aufgaben werden durch Vorrichtungen bzw. Verfahren mit den Merkmalen der unabhängigen Ansprüche gelöst. Vorteilhafte Ausführungsformen und Anwendungen der Erfindung ergeben sich aus den abhängigen Ansprüchen.

Gemäß einem ersten allgemeinen Gesichtspunkt der Erfindung wird die obige Aufgabe durch ein elektrophysiologisches Messgerät gelöst, das zur Erfassung mindestens eines elektrischen Messwerts an einer biologischen Zellprobe eingerichtet ist. Das elektrophysiologische Messgerät umfasst eine Substrateinrichtung, die zur Aufnahme der Zellprobe eingerichtet ist. Die Zellprobe umfasst eine einzelne biologische Zelle oder eine Vielzahl von Zellen (z. B. Zellhaufen, Zellaggregat, oder Gewebeabschnitt, insbesondere Zellaggregat mit durch Nexuskontakte verbundenen Zellen). Die Substrateinrichtung weist ein Substrat mit einer Substratoberfläche zur adhärenten Positionierung von Zellen auf. Die Substratoberfläche hat vorzugsweise eine ebene Form, wodurch eine Bezugsebene (im Folgenden auch: x-y-Ebene) des Substrats aufgespannt wird, wobei die Substratoberfläche selbst eben oder mit Erhebungen und/oder Vertiefungen strukturiert sein kann.

Des Weiteren ist die Substrateinrichtung zur Bereitstellung mindestens eines Flüssigkeitsfilms, vorzugsweise einer Flüssigkeitsschicht oder eines Flüssigkeitsbads, für eine Abdeckung der Zellen konfiguriert und hierzu vorzugsweise, jedoch nicht zwingend als Teil einer Kammer (Gefäß) gebildet. Die Kammer kann geschlossen (z. B. Schale) oder für einen Durchfluss eingerichtet sein (z. B. Kanal). Die Kammer kann oberhalb der Substrateinrichtung in einer Kammerwand einen Vorsprung zur Fixierung der Zellprobe aufweisen. Die Substrateinrichtung kann ein- oder mehrteilig sein und/oder zumindest teilweise durch Teile des Messgeräts gebildet werden, die für weitere Funktionen neben der Aufnahme der Zellprobe konfiguriert sind. Die Substrateinrichtung umfasst insbesondere ein Substrat, das in verschiedenen Konfigurationen einen einzigen Substratkörper zur Aufnahme der Zellprobe umfasst oder mit weiteren Komponenten, wie z. B. Membranen, kombiniert sein kann. Das Substrat hat eine Substratoberfläche, die vorzugsweise aus einem zellverträglichen Material hergestellt ist und z. B. eine zellverträgliche Beschichtung trägt. Die Substratoberfläche, insbesondere die Beschichtung erfüllt vorteilhafterweise eine Doppelfunktion in Bezug auf die Isolation der leitenden Spitze von mindestens einer Nanoelektrode und den direkten dichten Kontakt mit der Zellmembran.

Das elektrophysiologische Messgerät umfasst des Weiteren eine Elektrodeneinrichtung mit mindestens einer, vorzugsweise einer Vielzahl von Nanoelektroden (insbesondere mindestens zwei Nanoelektroden). Die Elektrodeneinrichtung weist eine einzige oder eine Vielzahl von Nanoelektroden auf. Werden im Folgenden Merkmale unter Bezug auf mehrere Nanoelektroden beschrieben, so sind diese Merkmale vorzugsweise in gleicher Weise auch bei Varianten mit einer einzigen Nanoelektrode gegeben. Jede Nanoelektrode erstreckt sich gerade in einer Longitudinalrichtung (im Folgenden auch: z-Richtung) von einem Elektrodenträger zu einer freien Elektrodenspitze (freies Ende). Der Durchmesser der Nanoelektroden, insbesondere an deren freien Elektrodenspitze, die in die Zellprobe ragt, ist kleiner als 1 µm, vorzugsweise kleiner oder gleich 800 nm, z. B. kleiner oder gleich 500 nm, und/oder mindestens 50 nm. Die longitudinale Länge der Nanoelektroden beträgt vorzugsweise mindestens 100 nm, besonders bevorzugt mindestens 1 µm, und/ oder höchstens 10 µm, besonders bevorzugt höchstens 3 µm.

Die Elektrodeneinrichtung und die Substrateinrichtung sind so angeordnet, dass die Nanoelektroden intrazellulär in die Zellprobe einführbar sind, wenn diese von der Substrateinrichtung aufgenommen ist. Die Nanoelektroden haben eine Konfiguration, dass sie in mindestens eine der Zellen der Zellprobe ragen können. Die Elektrodeneinrichtung und die Substrateinrichtung sind miteinander verbunden. Vorzugsweise sind die Nanoelektroden so angeordnet, dass die Longitudinalrichtung der Nanoelektroden senkrecht zu der Bezugsebene der Substratoberfläche verläuft. In mindestens einem Betriebszustand des Messgeräts sind die Nanoelektroden relativ zur Substratoberfläche so positioniert, dass die freien Elektrodenspitzen einen senkrechten Abstand von der Substratoberfläche haben, so dass die Nanoelektroden mit dem Zytosol im Zellinneren der Zellprobe auf der Substratoberfläche in direktem Kontakt sind. Der senkrechte Abstand von der Substratoberfläche beträgt vorzugsweise mindestens 1 µm und/oder höchstens 1 mm, kann aber auch im Bereich geringer als 1 µm gewählt sein. Die Nanoelektroden können auf einem Schaft aufsitzen und Säulen bilden oder sich konisch zu einer Spitze verjüngen. Der Schaft kann durch einen Zylinder oder Kegelstumpf mit einer Höhe von 1 µm bis 50 µm gebildet werden, auf den die Nanoelektroden aufgesetzt werden. Der Schaft kann jedoch auch höher sein.

Vorzugsweise ist die Elektrodeneinrichtung des elektrophysiologischen Messgeräts mit einer Messeinrichtung koppelbar oder fest verbunden. Die Messeinrichtung ist konfiguriert, elektrische Größen, insbesondere Spannungen und/oder Ströme, an den Nanoelektroden zu erfassen und elektrophysiologische Messwerte, wie z. B. Ruhepotentiale, Aktionspotentiale oder lonenströme durch Zellmembranen zu ermitteln. Vorzugsweise erfolgt die Messung von lonenströmen durch die Zellmembran ("patch-clamp"-Messung) oder die Messung von Aktionspotentialen an der Zellmembran ("current-clamp"-Messung). Es werden auch gleichzeitige oder aufeinanderfolgende Messungen sowohl von Aktionspotentialen als auch von lonenströmen mit der cell-attached patch clamp-Methode an einer Zellprobe ermöglicht.

Gemäß der Erfindung ist das elektrophysiologische Messgerät mit einer Stelleinrichtung ausgestattet, die für eine gegenseitige Bewegung der Nanoelektroden und/oder der Zellprobe entlang der Longitudinalrichtung der Nanoelektroden ausgelegt ist. Die gegenseitige Bewegung ist so gebildet, dass die Nanoelektroden in die Zellprobe eindringen können, wenn diese von der Substrateinrichtung aufgenommen ist. Die Stelleinrichtung wirkt auf die Nanoelektroden, insbesondere deren Elektrodenträger und/oder die Nanoelektroden selbst, und/oder die Substrateinrichtung, insbesondere das Substrat oder eine seitliche Kammerwand, so dass die Nanoelektroden zu der Zellprobe und/oder die Zellprobe zu den Nanoelektroden aktiv bewegt werden. Die Stelleinrichtung wirkt so, dass die Nanoelektroden gleichzeitig in die Zellprobe dringen. Die Relativbewegung erstreckt sich vorzugsweise über einen Weg im Bereich von 100 nm bis 5 mm.

Vorteilhafterweise werden durch die Bereitstellung der Stelleinrichtung des elektrophysiologischen Messgeräts zahlreiche Beschränkungen der herkömmlichen Techniken überwunden. Die Nanoelektroden, insbesondere deren Elektrodenspitzen, werden gezielt in der biologischen Zellprobe platziert. Die Einführung der Nanoelektroden in das Zytosol wird durch die Betätigung der Stelleinrichtung ausgelöst. Die Messung ist nicht von spontanen oder durch Elektrodenbeschichtungen erfolgende oder ausgelöste Prozesse abhängig. Die Genauigkeit, Reproduzierbarkeit und Zuverlässigkeit der Messung wird durch die Positionierung der Nanoelektroden in der Zellprobe verbessert. Die Erfinder haben festgestellt, dass biologische Zellproben die gleichzeitige Einführung einer Vielzahl von Nanoelektroden mit einer Penetration der Zellmembran erlauben, ohne dass die Nanoelektroden beschädigt werden. Indem die Stelleinrichtung eine gegenseitige Bewegung der Nanoelektroden und/oder der Zellprobe entlang der Longitudinalrichtung der Nanoelektroden erlaubt, werden unerwünschte Lateralkräfte, welche Nanoelektroden verbiegen könnten, vermieden oder auf ein vernachlässigbares Maß reduziert. Die Erfinder haben des Weiteren festgestellt, dass die Zellmembran stabilisierend auf die Nanoelektroden wirkt, so dass diese von der Zellmembran in das Zytosol geführt werden.

Gemäß einem zweiten allgemeinen Gesichtspunkt der Erfindung wird die obige Aufgabe durch ein elektrophysiologisches Messverfahren gelöst, bei dem mindestens ein elektrischer Messwert an einer biologischen Zellprobe intrazellulär erfasst wird. Das Messverfahren, das vorzugsweise mit dem elektrophysiologischen Messgerät gemäß dem ersten allgemeinen Gesichtspunkt der Erfindung ausgeführt wird, umfasst die folgenden Schritte. Zunächst wird die Zellprobe, umfassend eine einzelne Zelle oder eine Vielzahl von Zellen, auf einer Substrateinrichtung angeordnet. Die Anordnung der Zellprobe auf der Substrateinrichtung umfasst z. B. eine Positionierung einer Zellsuspension auf einem Substrat der Substrateinrichtung, optional gefolgt von einer Kultivierung der Zellprobe. Alternativ umfasst die Anordnung der Zellprobe eine Positionierung der Substrateinrichtung in einer Zellsuspension, gefolgt von einer Übertragung von mindestens einer Zelle auf die Substrateinrichtung. Anschließend oder bei der Übertragung der mindestens einen Zelle auf die Substrateinrichtung wird eine Vielzahl von Nanoelektroden einer Elektrodeneinrichtung so positioniert, dass die Nanoelektroden, insbesondere deren Elektrodenspitzen, intrazellulärer in die Zellprobe ragen. Die Nanoelektroden erstrecken sich parallel zueinander in einer Longitudinalrichtung und senkrecht zu einer Bezugsebene, welche eine laterale Ausdehnung der Substratoberfläche repräsentiert. Die Positionierung der Nanoelektroden umfasst gemäß der Erfindung eine Bewegung der Nanoelektroden und/oder der Zellprobe entlang der Longitudinalrichtung der Nanoelektroden mit einer Stelleinrichtung, bis die Nanoelektroden in die Zellprobe ragen und einen direkten elektrischen Kontakt mit dem Zellinneren bilden. Anschließend erfolgt die Messung des mindestens einen elektrischen Messwertes an der Zellprobe.

Die Penetration von Zellen der Zellprobe kann bei Betätigung der Stelleinrichtung so ablaufen, dass die Nanoelektroden mit ihren Spitzen zunächst die Zellmembran kontaktieren und deformieren (Destabilisierung) und anschließend durch eine pulsförmige Vorwärtsbewegung in die Zellen eindringen. Die pulsförmige Vorwärtsbewegung kann durch eine geeignete Steuerung der Stelleinrichtung, insbesondere Steuerung des Aktors durch die Antriebseinheit, bereitgestellt werden.

Die Bereitstellung der Stelleinrichtung bietet den Vorteil, dass die Zellprobe vor der Messung räumlich von den Nanoelektroden getrennt angeordnet sein kann. Es kann ein unbeabsichtigtes Eindringen der Nanoelektroden in die Zellen verhindert oder auf ein vernachlässigbares Maß vermindert werden. Teile des Messgeräts, insbesondere der Substrateinreichung und/oder der Stelleinrichtung bilden eine Trenneinrichtung, die zur räumlichen Trennung der Zellprobe von den Nanoelektroden vor Betrieb der Stelleinrichtung eingerichtet ist. Mit Betätigung der Stelleinrichtung wird ein zeitlicher Bezugspunkt für die elektrophysiologische Messung gesetzt, so dass diese insbesondere die Erfassung einer Zeitabhängigkeit von elektrophysiologischen Größen umfassen kann.

Vorteilhafterweise sind eine Reihe von Varianten möglich, die Stelleinrichtung im erfindungsgemäßen elektrophysiologischen Messgerät zu realisieren, wie im Folgenden dargestellt wird.

Gemäß der Erfindung umfasst die Stelleinrichtung als Aktor und Antriebseinheit eine Saugeinrichtung, die für ein Ziehen der Zellprobe zu den Nanoelektroden eingerichtet ist. Die Saugeinrichtung wird durch mindestens eine Saugöffnung und eine Pumpeinrichtung (Saugpumpe) gebildet, die über die mindestens eine Saugöffnung in der Substrateinrichtung mit dem Raum über der Substratoberfläche verbunden ist. Das Substrat ist mit der mindestens einen Nanoelektrode ausgestattet. Vorzugsweise sind die Nanoelektroden bei der ersten Ausführungsform auf dem Substrat fixiert, von dem Substrat vorragend oder in das Substrat versenkt angeordnet. Bei Beaufschlagung der mindestens einen Saugöffnung mit einem Unterdruck relativ zum umgebenden Atmosphärendruck mit der Pumpeinrichtung werden die Zellprobe und/oder eine umgebende Suspensionsflüssigkeit zum Substrat hin gezogen. Die mindestens eine Saugöffnung ist so gebildet, dass bei Beaufschlagung der mindestens einen Saugöffnung mit dem Unterdruck die Zellprobe zur Substratoberfläche und mit mindestens einer Richtungskomponente parallel zur Longitudinalrichtung der Nanoelektroden bewegt wird. Bei Berührung einer Nanoelektrode mit einer Zelle wird die Nanoelektrode durch die Saugbewegung in die Zelle geführt. Die erste Ausführungsform der Erfindung hat die besonderen Vorteile, dass das Messgerät einen einfachen Aufbau hat und die Stelleinrichtung einfach steuerbar ist. Die Leistung der Pumpeinrichtung ist vorzugsweise veränderlich und steuerbar, so dass ein vorbestimmter Ansaugvorgang ausgeführt werden kann, der eine zuverlässige Penetration der Membran der Zellprobe ermöglicht. Der Ansaugvorgang erfolgt beispielsweise derart, dass in einer ersten Phase mit einem geringeren Saugdruck eine langsamere Bewegung der Zellprobe bewirkt wird als in einer zweiten Phase mit einem höheren Saugdruck. Mit der Saugeinrichtung können Zellproben verschiedener Größe bewegt werden. Die Wegstrecke, die von der Zellprobe zurückgelegt wird, ist durch die Dauer des Betriebs der Pumpeinrichtung einstellbar. Mit der Saugeinrichtung sind auch nicht-adhärente Zellen im Raum oberhalb des Substrats erfassbar.

Gemäß der Erfindung ist oberhalb der Substrateinrichtung eine Kammer mit einer Kammerwand mit einer sich zum Substrat hin verengenden Konusform vorgesehen, wobei die Kammer zur Aufnahme eines Kultivierungsmediums und der Zellprobe in Gestalt eines Zellaggregats eingerichtet ist, und oberhalb der Substrateinrichtung ist in der Kammerwand am unteren Ende der Kammer ein Vorsprung zur Fixierung der Zellprobe vorgesehen.

Vorzugsweise sind bei der Erfindung die Nanoelektroden auf dem Substrat der Substrateinrichtung vorstehend angeordnet, wobei die mindestens eine Saugöffnung vorzugsweise unmittelbar benachbart zu den Nanoelektroden angeordnet ist. Mit anderen Worten, die Nanoelektroden sind außerhalb der mindestens einen Saugöffnung an deren Rand angeordnet (randständige Nanoelektroden). Vorzugsweise befinden sich die Nanoelektroden und die mindestens eine Saugöffnung in einer Ebene. Die Zellmembran einer Zellprobe wird nicht im Zentrum der Saugöffnung, sondern seitlich neben der Saugöffnung penetriert. Vorteilhafterweise wird durch die Anordnung am Rand der mindestens einen Saugöffnung die Bewegung der Zellprobe parallel zur Longitudinalrichtung der Nanoelektroden vereinfacht. Des Weiteren wird durch die Verwendung von randständigen Nanoelektroden die Herstellung des erfindungsgemäßen Messgeräts und insbesondere die Kontaktierung der Nanoelektroden vereinfacht.

Vorteilhafterweise kann die mindestens eine Saugöffnung an ihrem Rand, wo sich die Nanoelektrode oder Nanoelektroden befinden, mit einer adhäsiven Oberfläche ausgestattet sein, welche die Zelladhäsion fördert. An der adhäsiven Oberfläche des Umfangsrandes wird mit der Zellprobe ein Sealwiderstand (insbesondere im hohen Mega- oder Giga-Ohm-Bereich) gebildet. Damit werden die gleichzeitigen oder aufeinanderfolgenden Messungen von Aktionspotentialen und von lonenströmen an einer Zellprobe mit der "cell-attached patch clamp"-Methode verbessert. Durch die Verwendung von randständigen Nanoelektroden werden Beschränkungen herkömmlicher "cell attached"-Messungen behoben, und das Membranpotential kann direkt bestimmt und für die simultane patch clamp-Messung an der Zellprobe verwendet werden.

Besonders bevorzugt ist die mindestens eine Saugöffnung im Substrat zwischen den Nanoelektroden vorgesehen, so dass beim Ansaugen der Zellprobe die angrenzenden Nanoelektroden in die Zellprobe eindringen. Die Nanoelektroden können z. B. entlang eines Kreises um eine zentrale Saugöffnung angeordnet sein. Es ist beispielsweise eine Vielzahl von Saugöffnungen im Substrat zwischen den Positionen der Nanoelektroden vorgesehen. Ein weiterer Vorteil der Saugeinrichtung besteht darin, dass aus der fluidischen Mikrosystemtechnik Komponenten verfügbar sind, mit denen die Saugeinrichtung des elektrophysiologischen Messgeräts realisierbar ist.

Gemäß einer vorteilhaften Variante kann die mindestens eine Nanoelektrode zu der mindestens einen Saugöffnung hin geneigt angeordnet sein. Die Neigung kann Vorteile für die zuverlässige und deformationsfreie Einführung der Nanoelektrode(n) in die Zellprobe haben.

Vorzugsweise ist jede Nanoelektrode über eine Verbindungsleitung mit einer Messeinrichtung verbunden, wobei die Verbindungsleitung außerhalb der mindestens einen Saugöffnung angeordnet ist, an deren Rand die Nanoelektrode positioniert ist. Vorteilhafterweise wird damit der Aufbau des Messgeräts vereinfacht.

Die Form der Saugöffnung kann vorteilhafterweise in Abhängigkeit von der konkreten Anwendung der Erfindung gewählt werden. Die Saugöffnung kann zum Beispiel eine Kreisform oder eine eckige Form haben. Alternativ kann die Saugöffnung eine lang gestreckte Schlitzform haben. Gemäß einer weiteren vorteilhaften Variante der Erfindung umfasst die Saugöffnung alternativ oder zusätzlich eine Ringöffnung, die in der Substrateinrichtung angeordnet und mit der Pumpeinrichtung verbunden ist. Die Ringöffnung ist so angeordnet, dass sie entlang der Bezugsebene des Substrats mindestens eine Gruppe der Nanoelektroden umgibt. Vorteilhafterweise bietet die Ringöffnung die Möglichkeit, im Raum über dem Substrat eine vereinheitlichte Strömung hin zu den Nanoelektroden zu bilden.

Gemäß einer weiteren Abwandlung der Erfindung können die Nanoelektroden in mindestens einer der Saugöffnung(en) im Substrat angeordnet sein. Es kann mindestens eine Nanoelektrode in jeder Saugöffnung vorgesehen sein. In diesem Fall erstreckt sich beispielsweise jeweils in einer Saugöffnung eine einzelne Nanoelektrode oder mehrere Nanoelektroden mit der Longitudinalrichtung senkrecht zur Bezugsebene des Substrats. Bei Beaufschlagung der Saugöffnung mit einem Unterdruck wird die Zellprobe an die Saugöffnung gezogen, wobei die Elektrodenspitze in das Zellinnere der Zellprobe eindringt. Die Zellprobe kann teilweise in die Saugöffnung gezogen werden, wo das Eindringen der Nanoelektroden in die Zellprobe erfolgt. Die Positionierung der Nanoelektroden in mindestens einer der Saugöffnungen hat den besonderen Vorteil, dass die Nanoelektroden in den Saugöffnungen einen mechanischen Schutz erhalten. Des Weiteren wird die Bewegung der Zellprobe parallel zur Longitudinalrichtung der Nanoelektroden erleichtert.

Vorzugsweise erstreckt sich die mindestens eine Nanoelektrode parallel zu einer Innenwand der Saugöffnung in der Substrateinrichtung. Die Innenwand erstreckt sich bevorzugt senkrecht zur Bezugsebene des Substrats. Vorteilhafterweise wird damit eine Stabilisierung der Nanoelektroden bei der Penetration von Zellen erzielt.

Elektrodenspitzen der Nanoelektroden können über die Substratoberfläche vorragen oder in der Saugöffnung versenkt sein. Im letzteren Fall ist die mindestens eine Nanoelektrode so angeordnet ist, dass ihr freies Ende einen vorbestimmten senkrechten Abstand zur Oberfläche der Substrateinrichtung aufweist.

Gemäß einer weiteren vorteilhaften Variante kann die mindestens eine Saugöffnung frei von Referenzelektroden für die intrazelluläre Erfassung des mindestens einen elektrischen Messwerts sein. Vorteilhafterweise ist für die erfindungsgemäß vorgesehene cell-attached-Messung keine Referenzelektrode erforderlich, da das intrazelluläre Potential der Zellprobe als Bezugspotential für die Messung verwendet werden kann.

Die Saugeinrichtung kann bei den oben beschriebenen Varianten der Erfindung unmittelbar auf die Zellprobe wirken. Gemäß einer weiteren Abwandlung der ersten Ausführungsform kann die Wirkung auf eine deformierbare Trägermembran vorgesehen sein, die zur Aufnahme der Zellprobe eingerichtet ist. Die deformierbare Trägermembran ist Teil der Substrateinrichtung. Die Trägermembran, die beispielsweise eine Silikonmembran ist (z. B. ultra-dünne und hyper-elastische Silikonmembran, siehe z. B. [18]) oder aus Kollagen hergestellt ist, hat eine derart geringe Dicke, dass sie bei Betätigung der Saugeinrichtung von den Nanoelektroden durchdrungen werden kann. Die Dicke der Trägermembran ist beispielsweise im Bereich von 1 µm bis 30 µm gewählt. Die deformierbare Trägermembran ist mit einem Abstand über dem Substrat mit den Nanoelektroden so angeordnet, dass bei Beaufschlagung der mindestens einen Saugöffnung mit dem Unterdruck die deformierbare Trägermembran mit der Zellprobe zum Substrat hin bewegt wird. Es erfolgt eine Bewegung der Zellprobe mit der Trägermembran parallel zu der Longitudinalrichtung der Nanoelektroden. Die Nanoelektroden durchdringen die Trägermembran und die Zellmembran der Zellprobe.

Gemäß einer weiteren Abwandlung ist vorgesehen, dass die Substrateinrichtung einen Mündungsbereich einer Saugrohreinrichtung, wie zum Beispiel eine Pipettenspitze, umfasst (keine Ausführungsform der Erfindung). Der Mündungsbereich ist ein freies Ende der Saugrohreinrichtung, an dem diese eine Mündungsöffnung aufweist. Die Nanoelektroden sind an einem Umfangsrand der Mündungsöffnung vorragend angeordnet. Die Nanoelektroden können Isolationsschichten tragen, welche Elektrodenspitzen der Nanoelektroden frei lassen, oder sie können eine visköse Beschichtung tragen, oder sie können unbeschichtet sein. Die Longitudinalrichtung der Nanoelektroden verläuft parallel zur Axialrichtung der Saugrohreinrichtung. Die Saugöffnung wird bei dieser Variante durch die Mündungsöffnung der Saugrohreinrichtung gebildet. Vorteilhafterweise ermöglicht diese Variante die Kombination mit an sich bekannten Techniken zur elektrophysiologischen Untersuchung von Zellproben, insbesondere mit der an sich bekannten patch-clamp-Technik. Besonders bevorzugt ist daher im Inneren der Saugrohreinrichtung eine Messelektrode angeordnet, die für eine lonenstrommessung mit einem patch-clamp-Verstärker (Stromverstärker) eingerichtet ist.

Alternativ zur obigen Darstellung kann die zuletzt genannte Variante, bei der die Substrateinrichtung den Mündungsbereich insbesondere einer patch-clamp-Pipette bildet, allgemein als neue patch-clamp-Pipette beschrieben werden, die mit Nanoelektroden zur Zellpenetration ausgestattet ist (keine Ausführungsform der Erfindung). Entsprechend wird eine patch-clamp-Pipette mit Nanoelektroden an der Pipettenspitze beschrieben, wobei die patch-clamp-Pipette zur Verbindung mit einer Messeinrichtung konfiguriert ist.

Zusätzlich kann die Stelleinrichtung als Aktor und Antriebseinheit eine Elektroden-Antriebseinrichtung umfassen, die auf die Elektrodeneinrichtung wirkt und für eine Einführung der Nanoelektroden in die Zellprobe eingerichtet ist (keine Ausführungsform der Erfindung). Das Substrat, insbesondere der Substratkörper, weist eine Vielzahl von Elektrodenöffnungen auf, in denen jeweils eine der Nanoelektroden angeordnet ist. Die Antriebseinrichtung ist bei dieser Variante für einen mechanischen Vorschub der Nanoelektroden durch die Elektrodenöffnungen konfiguriert, bis die Elektrodenspitzen über die Substratoberfläche in die Zellprobe ragen. Alternativ kann die Elektrodeneinrichtung so mit der Substrateinrichtung verbunden sein, dass die Nanoelektroden im Raum über der Substratoberfläche angeordnet sind, wobei die Elektrodenspitzen mit einem Abstand von der Substratoberfläche angeordnet sind und zu der Substratoberfläche weisen. Die Elektroden-Antriebseinrichtung ist bei dieser Variante für einen mechanischen Vorschub der Nanoelektroden hin zu der Substratoberfläche konfiguriert, bis die Elektrodenspitzen in die Zellprobe auf der Substratoberfläche ragen. Diese Variante hat den Vorteil, dass mit der Stelleinrichtung die Nanoelektroden verstellt und in die ortsfest (adhärent) auf der Substrateinrichtung angeordnete Zellprobe eingeführt werden können. Mit der Antriebseinrichtung kann die Eindringtiefe der Nanoelektroden in die Zellprobe mit erhöhter Genauigkeit und Reproduzierbarkeit eingestellt werden.

Gemäß einer besonders bevorzugten Variante, bei der die Nanoelektroden substratseitig angeordnet sind, ist vorgesehen, dass das Substrat der Substrateinrichtung eine fixierte, formhaltige Porenmembran aufweist. Die Porenmembran bildet die Substratoberfläche zur Aufnahme der Zellprobe. Die Porenmembran weist Öffnungen auf, welche wie die Elektrodenöffnungen im Substratkörper angeordnet sind. Die Antriebseinrichtung ist so angeordnet, dass sie bei ihrer Betätigung die Elektrodeneinrichtung hin zu der Porenmembran drückt, wobei die Nanoelektroden durch die Öffnungen der Porenmembran in die Zellprobe vorgeschoben werden. Vorteilhafterweise sind die Adhäsionskräfte, welche die Zellprobe auf der Porenmembran festhalten, größer als die zur Penetration der Nanoelektroden in die Zellprobe erforderlichen Vortriebskräfte.

Wenn die Substrateinrichtung gemäß einer weiteren Abwandlung einen komprimierbaren Substratkörper umfasst, der zwischen der Elektrodeneinrichtung und der Porenmembran angeordnet ist, und die Elektroden-Antriebseinrichtung die Elektrodeneinrichtung gegen den Substratkörper drückt, so dass der Substratkörper zwischen der Elektrodeneinrichtung und der Porenmembran komprimiert wird, während die Nanoelektroden durch die Elektrodenöffnungen durch den Substratkörper und die Porenmembran in die Zellprobe vorgeschoben werden, können zusätzliche Vorteile hinsichtlich der Stabilisierung der Porenmembran bei der Betätigung der Elektroden-Antriebseinrichtung erzielt werden (keine Ausführungsform der Erfindung). Der Substratkörper ist aus einem komprimierbaren Kunststoff, wie z. B. Polydimethylsiloxan (PDMS), hergestellt.

Gemäß einer alternativen Abwandlung können die Elektrodenöffnungen in der Substrateinrichtung mit einer Deckelmembran verschlossen sein, wobei die Elektroden-Antriebseinrichtung für die Bewegung der Nanoelektroden durch die Deckelmembran in die Zellprobe eingerichtet ist. Vorteilhafterweise werden die Nanoelektroden durch die Deckelmembran von der Zellprobe getrennt. Die Deckelmembran bildet eine Variante einer Trenneinrichtung, mit der das elektrophysiologische Messgerät ausgestattet sein kann (keine Ausführungsform der Erfindung).

Es bestehen mehrere Möglichkeiten, die Nanoelektroden mit der Elektroden-Antriebseinrichtung mechanisch zu verstellen. Bevorzugt wird die Elektroden-Antriebseinrichtung durch die Nanoelektroden selbst gebildet, indem diese aus einem Material hergestellt werden, das in Reaktion auf eine äußere Stimulation eine Expansion zeigt. In diesem Fall bilden die Elektroden selbst den Aktor. Alternativ oder zusätzlich kann die Elektroden-Antriebseinrichtung durch den als Aktor wirkenden Elektrodenträger der Nanoelektroden gebildet werden. Die Elektroden-Antriebseinrichtung, insbesondere die Nanoelektroden und/oder der Elektrodenträger, ist z. B. für eine piezoelektrische, elektrische, mechanische und/oder magnetische Bewegung der Nanoelektroden eingerichtet. Die Nanoelektroden und/oder der Elektrodenträger können aus einem Material hergestellt sein, das für eine Expansion, insbesondere Längenänderung der Nanoelektroden ausgelegt ist, wie z. B. einem piezoelektrischen Material und/oder einem organischen Material, wie z. B. einem Polymer oder Harz. Als Antriebseinheit ist in diesen Fällen eine elektrische Ansteuerung der Aktoren vorgesehen.

Zusätzlich kann die Stelleinrichtung als Aktor und Antriebseinheit eine Substrat-Antriebseinrichtung umfassen, die mit der Substrateinrichtung gekoppelt und für ein Ziehen der Zellprobe zu den Nanoelektroden eingerichtet ist (keine Ausführungsform der Erfindung). In diesem Fall kann der Substratkörper selbst den Aktor bilden und aus einem Material hergestellt sein, das in Reaktion auf eine äußere Stimulation eine Kontraktion zeigt. Alternativ kann der Substratkörper mit einem Aktor verbunden sein, mit dem sich der Substratkörper verschieben lässt. Die Nanoelektroden sind ortsfest mit der Substrateinrichtung verbunden. Bei Stimulation des Substratkörpermaterials oder der Verschiebung des Substratkörpers wird die Zellprobe auf dem Substrat gegen die Nanoelektroden gezogen, so dass diese in die Zellprobe eindringen. Diese Variante hat den Vorteil, dass mit der Stelleinrichtung der Substratkörper verstellt und zu den ortsfesten Nanoelektroden bewegt werden kann. Dies ermöglicht eine zusätzliche mechanische Stabilisierung der Nanoelektroden, z. B. mit einer partiellen Einbettungsschicht, und kann die Verbindung der Nanoelektroden mit der Messeinrichtung vereinfachen.

Zusätzlich kann die Stelleinrichtung als Aktor und Antriebseinheit eine Magnet-Antriebseinrichtung umfassen, die als Aktor magnetische Partikel, die für eine Kopplung mit der Zellprobe vorgesehen sind, und als Antriebseinheit einen Hauptmagneten umfasst, der für eine Wechselwirkung mit den magnetischen Partikeln und ein Ziehen der Zellprobe zu den Nanoelektroden angeordnet ist (keine Ausführungsform der Erfindung). Vorteilhafterweise sind aus bekannten Techniken der Zellbiologie magnetische Partikel (magnetische Beads) verfügbar, welche inert und mit biologischen Zellen koppelbar sind. Ein weiterer Vorteil dieser Variante besteht darin, dass die Substrateinrichtung und die Elektrodeneinrichtung ortsfest ohne bewegliche Teile gebildet sein können, wodurch sich der Aufbau des Messgeräts vereinfacht. Schließlich kann es von Vorteil sein, bei der Messung einer Zellprobe mit verschiedenen Zelltypen, von denen nur eine bestimmte Gruppe interessierender Zellen mit den magnetischen Partikeln ausgestattet ist, selektiv nur die interessierenden Zellen zu den Nanoelektroden zu bewegen.

Zusätzlich kann die Stelleinrichtung als Aktor und Antriebseinheit eine Kammer-Antriebseinrichtung umfassen, die mit einer mit der Substrateinrichtung verbundenen Kammerwand gekoppelt und für ein Ziehen der Zellprobe zu den Nanoelektroden eingerichtet ist (keine Ausführungsform der Erfindung). In diesem Fall ist die Kammerwand als Aktor aus einem Material hergestellt, das in Reaktion auf eine äußere Stimulation eine Kontraktion zeigt. Die Elektrodeneinrichtung ist so über die Kammerwand mit der Substrateinrichtung verbunden, dass die Nanoelektroden im Raum über der Substratoberfläche angeordnet sind, wobei die Elektrodenspitzen mit einem Abstand von der Substratoberfläche angeordnet sind und zu der Substratoberfläche weisen. Durch eine Kontraktion oder mechanische Kompression der Kammerwand werden die Nanoelektroden hin zu der Substratoberfläche bewegt, bis die Elektrodenspitzen in die Zellprobe auf der Substratoberfläche ragen. Diese Variante hat den Vorteil, dass mit der Stelleinrichtung die Nanoelektroden verstellt und in die ortsfest (adhärent) auf der Substrateinrichtung angeordnete Zellprobe eingeführt werden können. Mit der Kontraktion oder Kompression der Kammerwand kann die Eindringtiefe der Nanoelektroden in die Zellprobe mit erhöhter Genauigkeit und Reproduzierbarkeit eingestellt werden. Die Bewegung kann dabei durch Anschlagselemente beschränkt werden.

In einem praktischen Messverfahren können Zellen, wie z. B. enzymatisch isolierte Herzmuskelzellen, enzymatisch isolierte neuronale Zellen, aus Stammzellen differenzierte Herzzellen oder aus Stammzellen differenzierte neuronale Zellen auf einem Nanoelektroden-Array kultiviert werden. Ein erster Schritt kann in einem Beschichten der Substratoberfläche (Kulturoberfläche) mit Matrigel oder Fibronektin bestehen, was die Haftung verbessert und auch Differenzierungsprozesse anregen kann. Die Beschichtung kann alternativ bei der Herstellung erfolgen (Verwendung beschichteter Nanoelektroden-Arrays). Nach Zugabe der Zellen haften diese auf der Substratoberfläche, die optional strukturiert ist und Nuten oder Nuten mit seitlichen Ankerelementen aufweisen kann.

Die Penetration der Nanoelektroden in die Zellprobe kann durch Elektroporation gleichzeitig zur Betätigung der Stelleinrichtung unterstützt werden. Dabei ist der Durchmesser der leitfähigen Spitze vorzugsweise kleiner oder gleich 500 nm, insbesondere kleiner oder gleich 200 nm, z. B. kleiner oder gleich 100 nm, und/oder kegelförmig, wodurch eine fokussierte Applikation des Membranpotentials ermöglicht und eine Penetration (Durchschlag) der Membran erleichtert wird.

Bei dem elektrophysiologischen Messgerät gemäß allen Ausführungsformen können die Nanoelektroden eine elektrisch isolierende, visköse Beschichtung tragen, die sich bei einer Einführung der Nanoelektroden in die Zellprobe von den Nanoelektroden durch die Wirkung der Zellmembran abstreifen lässt. Die Verwendung der viskösen Beschichtung hat den Vorteil, dass sie sich relativ einfach auftragen und bei Anwendung des Messgeräts leicht entfernen lässt.

Weitere Einzelheiten und Vorteile der Erfindung werden im Folgenden unter Bezug auf die beigefügten Zeichnungen beschrieben. Es zeigen schematisch:
- Figuren 1 bis 6:: Varianten von elektrophysiologischen Messgeräten (keine Ausführungsformen der Erfindung);
- Figur 7:: ein elektrophysiologisches Messgerät gemäß einer Ausführungsform der Erfindung;
- Figuren 8 bis 18:: weitere Varianten von elektrophysiologischen Messgeräten (keine Ausführungsformen der Erfindung);
- Figur 19:: ein elektrophysiologisches Messgerät gemäß einer Ausführungsform der Erfindung;
- Figuren 20 bis 21:: weitere Varianten von elektrophysiologischen Messgeräten (keine Ausführungsformen der Erfindung).

Ausführungsformen der Erfindung werden im Folgenden unter beispielhaftem Bezug auf ein elektrophysiologisches Messgerät beschrieben, das mit bis zu zwanzig Nanoelektroden ausgestattet ist. Die Erfindung ist nicht auf diese Beispiele beschränkt, sondern entsprechend mit einem Messgerät oder Hochdurchsatz-Testgerät realisierbar, das eine größere Anzahl von Nanoelektroden aufweist, die als eine einzelne Reihe oder als zweidimensionales Array angeordnet sind. Es können beispielweise in einem Messgerät mehr als 500 Nanoelektroden (oder bis zu 1000), die jeweils mit mindestens einer Messeinrichtung koppelbar sind, vorgesehen sein, so dass eine Vielzahl von Zellproben gleichzeitig bereitgestellt und gleichzeitig oder aufeinanderfolgend untersucht werden können. Es kann auch eine einzige Nanoelektrode vorgesehen sein.

Teile des Messgeräts, die z. B. als Substrateinrichtung oder als Elektrodeneinrichtung bezeichnet werden, können durch getrennte oder gemeinsame Komponenten realisiert werden. Beispielsweise kann die Elektrodeneinrichtung zumindest teilweise die Substrateinrichtung oder die Stelleinrichtung zumindest teilweise eine Kammer des Messgeräts bilden.

Des Weiteren wird beispielhaft auf Messungen von lonenströmen und/oder Aktionspotentialen und/oder auf patch-clamp-Messungen Bezug genommen. Hierzu weisen Saugöffnungen, die bei Ausführungsformen der Erfindung vorgesehen sind, vorzugsweise an ihren Rändern eine adhäsive Oberflächenbeschichtung zur Unterstützung der Bildung des Sealwiderstandes auf. Die Erfindung ist nicht auf diese Anwendung beschränkt, sondern entsprechend mit anderen elektrophysiologischen Messungen, wie z. B. der Messung von Bewegungen mit Hilfe von elektrischem Zell-Substrat-Sensing (siehe z. B. [18, 19]), oder in Kombination mit weiteren Messungen, wie z. B. Fluoreszenz-Messungen oder der Erfassung von intrazellulärem Kalzium oder anderen Ionen, ausführbar. Einzelheiten von elektrophysiologischen Messungen, wie die Auswahl von elektrischen Messparametern, die Ableitung elektrophysiologischer Messsignale von den Nanoelektroden, die Anordnung von Nanoelektroden, die Verstärkertechnik und/oder die Auswahl von Perfusionsmedien, Einzelheiten der Probenpräparation und Einzelheiten der Handhabung und ggf. Kultivierung von biologischen Zellproben, wie z. B. die Herstellung von Zellaggregaten, werden nicht beschrieben, da diese an sich von herkömmlichen Techniken bekannt sind.

Die Erfindung wird insbesondere in Bezug auf die Konfiguration und den Betrieb der Stelleinrichtung und/oder die Konfiguration einer Substrateinrichtung mit einer strukturierten Oberfläche beschrieben. Das Messgerät oder ein Hochdurchsatz-Testgerät kann weitere Komponenten, wie zum Beispiel Flüssigkeitsreservoire, eine Fluidikeinrichtung zur Medienzu- und -abfuhr zum Substrat, Steuerelemente (z. B. steuerbare Ventile), Temperierungseinrichtungen, Befeuchtungseinrichtungen und dergleichen aufweisen. Ein erfindungsgemäßes Messgerät kann insbesondere ein autarkes Gerät für Labor- oder Industrieanwendungen zur Untersuchung von Zellproben sein. Alternativ kann das Messgerät Teil eines Kultivierungsgerätes für biologische Zellen sein, das insbesondere für die Kultivierung von Zellaggregaten ausgelegt ist. Des Weiteren kann eine Steuereinrichtung vorgesehen sein, mit der das Messgerät, insbesondere die Elektrodeneinrichtung und/oder die Stelleinrichtung, steuerbar sind.

Bei den beschriebenen Ausführungsformen sind die Nanoelektroden vorzugsweise mit einer elektrisch isolierenden Schicht, z.B. SiO₂, beschichtet, die Elektrodenspitzen der Nanoelektroden frei lässt, oder vollständig mit einer viskösen Substanz beschichtet, die beim Eindringen in die Zelle leitende Elektrodenspitzen der Nanoelektroden frei gibt, ohne dass dies bei der Beschreibung der einzelnen Figuren jeweils erwähnt wird. Des Weiteren können die Zellkulturflächen optional mit Matrigel oder Fibronektin beschichtet sein. Elastische Materialien können z. B. PDMS oder Gummi umfassen.

Richtungen werden im Folgenden in Bezug auf die Ausdehnung des Substrats (Bezugsebene) genannt. Richtungen parallel zur Bezugsebene werden auch als seitliche Richtungen bezeichnet. Die Begriffe "oben" und "unten" und davon abgeleitete Bezeichnungen verweisen auf die Richtungen hin zu den Deckel- bzw. Bodenabschnitten des Messgeräts. Die Zeichnungen sind schematische Schnittansichten, die teils zur Verdeutlichung durch perspektivisch dargestellte Abschnitte ergänzt sind.

Die Zeichnungen sind schematische Illustrationen. In der Praxis können die Größenverhältnisse und Formen der Teile des Messgeräts von den Illustrationen abweichend gewählt sein. Beispielsweise kann die Kammer zur Aufnahme der Zellprobe eine Form aufweisen, wie in [16] beschrieben ist. [16] wird insbesondere hinsichtlich der Formen der Kammer zur Aufnahme der Zellprobe durch Bezugnahme in die vorliegende Offenbarung einbezogen.

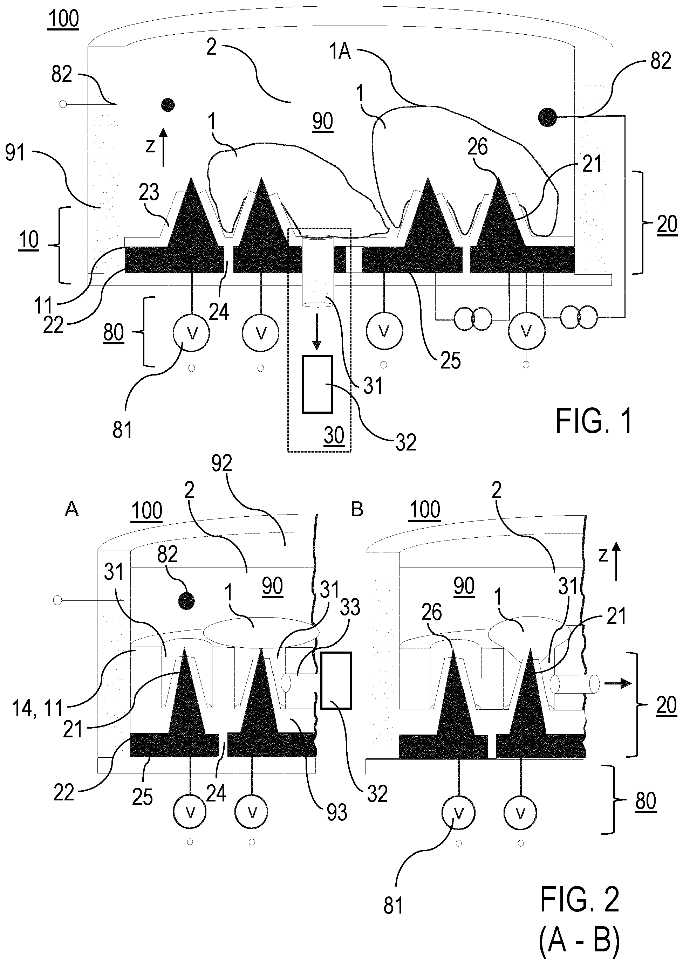

Figur 1 zeigt eine erste Variante eines elektrophysiologischen Messgeräts 100 mit einer Substrateinrichtung 10 und einer Elektrodeneinrichtung 20 (keine Ausführungsform der Erfindung). Die Substrateinrichtung 10 umfasst ein Substrat 11, das zur Aufnahme der Zellprobe 1 vorgesehen ist. Das Substrat 11 erstreckt sich in einer Bezugsebene, die in Figur 1 im Wesentlichen senkrecht zur Zeichenebene ausgerichtet ist. Des Weiteren ist die Substrateinrichtung 10 Teil einer Kammer 90, deren Kammerwand 91 im Raum über dem Substrat 11 ein Gefäß zur Aufnahme eines Kultivierungsmediums 2 bildet.

Die Elektrodeneinrichtung 20 umfasst eine Vielzahl von Nanoelektroden 21, die auf einem ebenen Elektrodenträger 22 angeordnet sind. Die Nanoelektroden 21 erstrecken sich in einer Longitudinalrichtung (parallel zur z-Richtung) senkrecht zur Bezugsebene des Substrats 11. Das Substrat 11 wird bei dieser Ausführungsform der Erfindung durch den Elektrodenträger 22 und eine Isolationsschicht 23, gebildet, die den Elektrodenträger 22 bedeckt. Die Isolationsschicht 23 lässt Elektrodenspitzen 26 der Nanoelektroden 21 frei, so dass ein direkter elektrischer Kontakt zwischen den Nanoelektroden 21 und dem Zytosol in der Zellprobe 1 gebildet wird. Die Isolationsschicht 23, die eine Verbindung mit der Zellmembran 1A (Lipidmembran) bildet, besteht z. B. aus SiO₂ mit einer Dicke von 10 nm bis 100 nm.

Alternativ können die Nanoelektroden allgemein aus einem nicht-leitenden Material, wie z. B. einem Kunststoff, gebildet sein und eine leitfähige Beschichtung (10 - 100 nm) aus einem leitenden Material, z.B. Gold, tragen. Auf der leitfähigen Beschichtung kann eine Isolationsschicht, z. B. aus SiO₂, mit einer Dicke im Bereich von 10 nm bis 100 nm vorgesehen sein.

Die Nanoelektroden 21 und voneinander elektrisch isolierte Trägerabschnitte 25 des Elektrodenträgers 22 sind aus einem elektrisch leitfähigen Material, wie zum Beispiel Gold, Platin, Iridium, Iridiumoxid und Rhodiumoxid, Titanid, Silber/Silberchlorid hergestellt. Die Nanoelektroden 21 haben eine Kegel- oder Pyramidenform mit einer longitudinalen Länge von z. B. 100 nm bis 10 µm und einem Durchmesser an den Elektrodenspitzen von z. B. 50 nm bis 200 nm. Der Elektrodenträger 22 weist Isolationsabschnitte 24 auf, die den Elektrodenträger 22 in die Vielzahl elektrisch isolierter, elektrisch leitfähiger Trägerabschnitte 25 unterteilt. Jeder der Trägerabschnitte 25 ist mit einer der Nanoelektroden 21 elektrisch verbunden. Des Weiteren ist jeder Trägerabschnitt 25 jeweils mit einer Messschaltung 81 einer Messeinrichtung 80 verbunden. Die Messeinrichtung 80 weist ferner Referenzelektroden 82 auf, die in der Kammer 90 über dem Substrat 11 angeordnet und mit dem Kultivierungsmedium 2 in der Kammer 90 elektrisch verbunden sind. Die Messschaltungen 81 sind zur Messung elektrischer Spannungs- und/oder Stromwerte an der Zellprobe 1 vorgesehen. Die Messeinrichtung 80 ist des Weiteren optional mit Pulsgeneratoren zur Beaufschlagung der Zellprobe mit Spannungsimpulsen ausgestattet.

Bei dem in Figur 1 gezeigten elektrophysiologischen Messgerät 100 (keine Ausführungsform der Erfindung) wird eine Stelleinrichtung zur Relativbewegung der Zellprobe 1 und der Nanoelektroden 21 durch eine Saugeinrichtung 30 gebildet, die eine Saugöffnung 31 in der Substrateinrichtung 10 und eine Pumpeinrichtung 32 (Antriebseinheit) umfasst. Mit der Pumpeinrichtung 32, umfassend z. B. eine Mikroliterspritze (Hersteller Hamilton), kann die Saugöffnung 31 mit einem Unterdruck beaufschlagt werden, unter dessen Wirkung das Kultivierungsmedium 2 und mit diesem die Zellprobe 1 zur Saugöffnung 31 gezogen wird. Bei der Bewegung hin zu den Nanoelektroden 21 wird die Zellmembran 1A von Zellen der Zellprobe 1 penetriert, so dass die Elektrodenspitzen 26 in das Innere der Zellen der Zellprobe 1 ragen. Im Übrigen sind die Zellen von dem Elektrodenträger 22 durch die Isolationsschicht 23 elektrisch getrennt.

Obwohl in Figur 1 nur eine Saugöffnung 31 gezeigt wird, ist die Realisierung eines Messgeräts nicht auf diese Variante beschränkt. Es können mehr Saugöffnungen 31 vorgesehen sein, die im Substrat 11 zwischen den Nanoelektroden 21 verteilt angeordnet sind. Alle Saugöffnungen sind mit einer gemeinsamen Pumpeinrichtung 32 verbunden. Bei Betätigung der Pumpeinrichtung 32 werden Zellen in mehreren Abschnitten des von der Kammer 90 gebildeten Behälters gleichmäßig zu den Nanoelektroden 21 gezogen.

Bei einem Messverfahren zur intrazellulären Erfassung mindestens eines elektrischen Messwertes an der biologischen Zellprobe 1 werden die folgenden Schritte ausgeführt. Zunächst wird eine Suspension des Kultivierungsmediums 2, mit der Zellprobe 1 in die Kammer 90 gefüllt. Die Zellprobe 1, die zum Beispiel aus Stammzellen differenzierte Herzzellen umfasst, kann sich unter der Wirkung der Gravitation auf dem Substrat 11 absetzen, wobei jedoch noch keine oder nur eine vernachlässigbare Penetration der Zellmembran 1A durch die Nanoelektroden 21 erfolgt. Durch Betätigung der Pumpeinrichtung 32 wird die Zellprobe 1 zum Substrat 11 gesaugt, so dass die Nanoelektroden 21 in das Innere der Zellen der Zellprobe 1 eindringen. In einzelne Zellen können jeweils eine einzige Nanoelektrode oder mehrere Nanoelektroden 21 eindringen. Wieviel Nanoelektroden 21 in jeweils eine Zelle eingedrungen sind, kann z. B. durch eine elektrische Messung des Widerstands zwischen benachbarten Nanoelektroden 21 festgestellt werden. Anschließend erfolgt in an sich bekannter Weise eine elektrophysiologische Messung mit der Messeinrichtung 80. Die elektrophysiologische Messung kann an allen Nanoelektroden 21 gleichzeitig oder zeitlich aufeinanderfolgend erfolgen. Es kann eine Zeitabhängigkeit von Messwerten aufgenommen werden, z. B. um die Ausbreitung eines Aktionspotentials von Zelle zu Zelle in einer Zellprobe 1 zu messen.

Nach Abschluss der Messung kann die Zellprobe 1 aus der Kammer 90 entfernt werden. Alternativ kann anschließend eine Kultivierung der Zellprobe 1 in der Kammer 90 vorgesehen sein. Hierzu kann dem Kultivierungsmedium 2 weiteres Medium, umfassend zum Beispiel Nährstoffe und oder Differenzierungsfaktoren, zugesetzt werden. Nach einer vorbestimmten Kultivierungszeit kann mindestens eine weitere elektrische Messung an der Zellprobe 1 erfolgen.

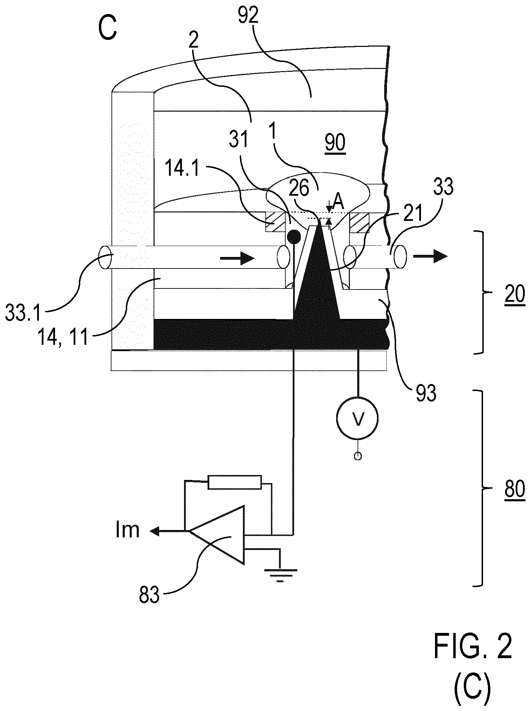

Figur 2 zeigt eine abgewandelte Variante des elektrophysiologischen Messgeräts 100 in verschiedenen Phasen der Relativbewegung der Zellprobe 1 und der Nanoelektroden 21 (keine Ausführungsform der Erfindung). Bei dieser Variante wird das Substrat 11 durch einen Substratkörper 14, z. B. aus PDMS oder Polyvinylchlorid (PVC) mit einer Dicke von 3 mm bis 5 mm, mit durchgehenden Saugöffnungen 31 gebildet, welche sich in z-Richtung senkrecht zur Bezugsebene des Substrats 11 erstrecken. Die Saugöffnungen 31 sind z. B. rund mit einem Durchmesser z. B. im Bereich von 1 bis 4 µm. Die Kammer 90 wird durch das Substrat 11 in einen oberen Kammerabschnitt 92 und einen unteren Kammerabschnitt 93 unterteilt, welche über die Saugöffnungen 31 miteinander verbunden sind. Eine der Saugöffnungen 31 ist mit einer schematisch gezeigten seitlichen Saugleitung 33 gezeigt, die mit der Pumpeinrichtung 32 (siehe Figur 2A) verbunden ist. In der Praxis kann jede der Saugöffnungen 31 mit einer seitlichen Saugleitung 33 ausgestattet sein und/oder mindestens eine Saugleitung mit dem unteren Kammerabschnitt 93 verbunden sein. Zusätzlich kann an wenigstens einer der Saugöffnungen 31 eine Perfusionsleitung 33.1 vorgesehen sein, wie schematisch in Figur 2C gezeigt ist.

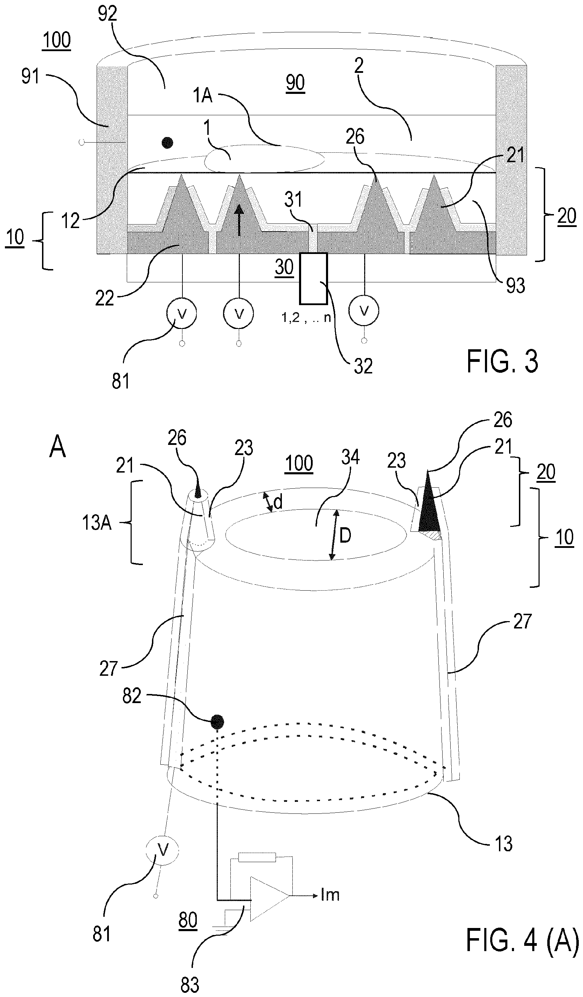

Die Saugöffnung 31 kann optional mit einer ultradünnen elastischen Folie (z.B. aus Silikon) abgedeckt sein, wie in Figur 3 dargestellt. Dies bietet den Vorteil, dass der Durchmesser der Saugporen vergrößert werden kann (in den Bereich von 50 µm bis 1 mm, sowie bis 10 mm), wobei verhindert wird, dass die Zellen in die Saugöffnung eintreten. Weitere Vorteile dieser Ausführungsform werden mit Bezug auf Figur 3 genannt.

Die Nanoelektroden 21 der Elektrodeneinrichtung 20 sind ortsfest im unteren Kammerabschnitt 93 angeordnet. Die Nanoelektroden 21 erstrecken sich in z-Richtung durch die Saugöffnungen 31. Die Nanoelektroden 21 sind in den Saugöffnungen 31 versenkt angeordnet. Vorzugsweise sind die Nanoelektroden 21 jeweils mittig in den Saugöffnungen 31 positioniert. Der Elektrodenträger 22 der Nanoelektroden 21 ist, wie oben unter Bezug auf Figur 1 beschrieben ist, durch Isolationsabschnitte 24 in einzelne Trägerabschnitte 25 unterteilt. Jeder der Trägerabschnitte 21 ist mit einer Messschaltung 81 der Messeinrichtung 80 elektrisch verbunden.

Bei Beaufschlagung der Saugleitung 33 mit einem Unterdruck wird das Kultivierungsmedium 2 insbesondere aus dem oberen Kammerabschnitt 92 durch die Saugöffnungen 31 gezogen. Gemeinsam mit der dabei auftretenden Flüssigkeitsbewegung werden einzelne Zellen oder Zellgruppen der Zellprobe 1 an die Saugöffnungen 31 gezogen. Nach dem Ansaugen der Zellprobe 1 auf die Saugöffnung 31 wird bei fortgesetzter Beaufschlagung der Saugleitung 33 mit dem Unterdruck die Zellprobe 1 teilweise in die Saugöffnung 31 gezogen, wie schematisch in den Figuren 2B und 2C gezeigt ist. Dabei trifft die Zellprobe 1 auf die Elektrodenspitze 26 der Nanoelektrode 21. Unter der Wirkung des Ansaugdruckes kommt es zur Penetration der Zellprobe 1 mit der Nanoelektrode 21. Anschließend erfolgt die Erfassung elektrischer Messwerte an der Zellprobe 1.

Das Messgerät 100 gemäß Figur 2 (keine Ausführungsform der Erfindung) kann gemäß einem oder mehreren der folgenden Merkmale abgewandelt realisiert werden. Es können mehrere Nanoelektroden 21 pro Saugöffnung 31 vorgesehen sein. Die Saugöffnungen 31 können eine andere Form, z. B. als Rille im Substratkörper, aufweisen. Die Saugöffnungen 31 können im Substratkörper nach unten hin geschlossen und jeweils mit einer seitlichen Saugleitung ausgestattet sein, wobei die Nanoelektroden fest im Substratkörper eingebettet sind. Es können Beschichtungen der Nanoelektroden und/oder der durch die Oberfläche des Substrates 11 gebildeten Kulturfläche vorgesehen sein.

Die Figuren 2A und 2B zeigen eine Anwendung des Messgeräts für ausschließliche Membranpotentialmessungen mit einer Referenzelektrode 82 im Bad (Kultivierungsmedium 2). Figur 2C zeigt eine abgewandelte Anwendung des Messgeräts für patch-clamp-Messungen mit fixierter Zelle ("cell attached"-Messung) mit einer Masseelektrode 82.1 in Flüssigkeitskontakt mit dem unteren Kammerabschnitt 93.

Insbesondere für die patch-clamp-Messung kann es von Vorteil sein, am oberen, zum oberen Kammerabschnitt 92 weisenden Ende der Saugöffnungen 31 auf dem Substrat 11 jeweils Adhäsionsabschnitte 14.1 vorzusehen. Die Adhäsionsabschnitte 14.1 (schematisch schraffiert gezeigt) umgeben jeweils den oberen Rand einer Saugöffnung 31. Adhäsionsabschnitte 14.1 können auch bei den Varianten gemäß den Figuren 2A und 2B (keine Ausführungsformen der Erfindung) und bei den Ausführungsformen der Erfindung vorgesehen sein.

Mit den Adhäsionsabschnitten 14.1 wird ein hochohmiger Kontakt (> 100 Megaohm) oder ein Giga-Seal am Rand der Saugöffnungen 31 gebildet. Damit wird die cell-attached Messung an der die Nanoelektrode 21 umgebenden Zellmembran bezüglich des mit der Nanoelektrode 21 gemessenen Membranpotentials durchgeführt. Beide Leitungen für Potential (V) und Membranstrom (Im) werden voneinander isoliert geführt und können mit der Messeinrichtung 80, umfassend einen patch-clamp-Verstärker 83, simultan gemessen werden.

In Figur 2C ist die Penetration der Zelle 1 nach Anlegen eines Unterdrucks mittels der Saugleitung 33 und/oder des unteren Kammerabschnitts 92 dargestellt, wobei der hochohmige Kontakt oder Gigaseal-Kontakt zum Substrat 11 gebildet wird. Dadurch ist die in Figur 2A gezeigte Referenzelektrode nicht erforderlich.

Die mindestens eine Nanoelektrode 21 hat eine mittige Position in der der Saugöffnung 31, die auch als Perfusionspore bezeichnet wird. Die Saugöffnung 31 bildet gleichzeitig um die jeweilige mindestens eine Nanoelektrode 21 ein perfundierbares Kompartiment. In diesem ist die Masseelektrode 82.1 zur Bildung einer zusätzlichen virtuellen Masse elektrisch isoliert von der Nanoelektrode 21 angeordnet. Die Masseelektrode 82.1 wird ebenfalls für die "cell attached" patch-clamp-Messungen verwendet.

Figur 2C illustriert ein weiteres vorteilhaftes Merkmal eines Messgeräts (keine Ausführungsform der Erfindung), das bei der Variante der Figur 2C und auch bei den beschriebenen Ausführungsformen optional vorgesehen sein kann. Die Nanoelektrode 21 hat einen Spitzenabstand A von der Oberfläche des Substrates (Abstand der Elektrodenspitze in Longitudinalrichtung der Nanoelektrode 21 von der Oberfläche, siehe Pfeil). Der Spitzenabstand A ist vorzugsweise im Bereich von 100 nm bis 5 µm gewählt. Der Durchmesser der Saugöffnung 31 ist für Messungen an Einzelzellen vorzugsweise im Bereich von 1,5 µm bis 5 µm und für Messungen an Zellaggregaten im Bereich von 10 µm bis 100 µm, insbesondere 10 µm bis 20 µm gewählt.

Die in Figur 3 gezeigte Variante des elektrophysiologischen Messgeräts 100 ist ähnlich zu dem Messgerät 100 gemäß Figur 1 aufgebaut (keine Ausführungsform der Erfindung). Die Substrateinrichtung 10 bildet mit der Kammer 90 ein Gefäß zur Aufnahme des Kultivierungsmediums 2 mit der Zellprobe 1. Die Substrateinrichtung 10 ist mit einer flexiblen deformierbaren Trägermembran 12 ausgestattet, die zur Aufnahme der Zellprobe 1 und optional zum Wachstum der Zellen der Zellprobe 1 vorgesehen ist. Mit der Trägermembran 12 wird die Kammer 90 in einen oberen Kammerabschnitt 92 und ein unteren Kammerabschnitt 93 unterteilt. Die Trägermembran 12 ist zum Beispiel eine Silikonmembran, beispielsweise mit einer Dicke im Bereich von 1 bis 5 µm. Die Trägermembran wirkt vorteilhafterweise gleichzeitig als Isolator, indem sie den Schaft der Nanoelektrode fest umschließt. Die ermöglicht die optionale Verwendung von nicht mit einer Isolationsschicht 23 beschichteten Nanoelektroden. Der Schaft der Nanoelektrode kann für diese Anwendung, z. B. auf bis zu 50 µm, verlängert werden.

Die Elektrodeneinrichtung 20 wird durch ortsfeste Nanoelektroden 21 in Verbindung mit einem ebenen Elektrodenträger 22 am Boden der Kammer 90 gebildet. Die Elektrodeneinrichtung 20 bildet ein Bodenteil, mit dem die Kammer 90 nach unten verschlossen ist. Die Trägermembran 12 ist zwischen den Wänden 91 der Kammer 90 aufgespannt, so dass sie sich parallel zur Ausdehnung des Elektrodenträgers 22 erstreckt. Die Trägermembran 12 kann mit einem Abstand von den Elektrodenspitzen 26 der Nanoelektroden 21 angeordnet sein oder diese berühren.

Die Stelleinrichtung zur Relativbewegung der Zellprobe 1 und der Nanoelektroden 21 umfasst die Saugeinrichtung 30 mit der mindestens einen Saugöffnung 31 im Bodenteil des Substrats 11 und eine mit der Saugöffnung 31 verbundene Pumpeinrichtung 32. Bei Beaufschlagung der Saugöffnung 31 mit einem Unterdruck wird Flüssigkeit aus dem unteren Kammerabschnitt 93 abgesaugt, so dass die Trägermembran 12 mit der Zellprobe 1 zur Elektrodeneinrichtung 20 und insbesondere zu den Nanoelektroden 21 gezogen wird. Die Nanoelektroden 21 durchstoßen die Trägermembran 12 und die Zellmembran 1A der Zellen der auf der Trägermembran 12 befindlichen Zellprobe 1, bis die Elektrodenspitzen zu 26 im Inneren der Zellen positioniert sind. Anschließend erfolgt die gewünschte elektrophysiologische Messung.

Die Trägermembran 12 wird durch eine Trenneinrichtung bereitgestellt, welche den Raum zur Aufnahme der Zellprobe 1 (oberer Kammerabschnitt 92) von den Nanoelektroden 21 im unteren Kammerabschnitt 93 trennt, solange die Saugeinrichtung 30 nicht betätigt und die Trägermembran 12 nicht zu den Nanoelektroden 21 gezogen wird.

Das Messgerät 100 gemäß den Figuren 1 bis 3 kann mit an sich verfügbaren Herstellungsverfahren der fluidischen Mikrosystemtechnik aus Kunststoff, metallischen Komponenten und/oder Keramik hergestellt werden.

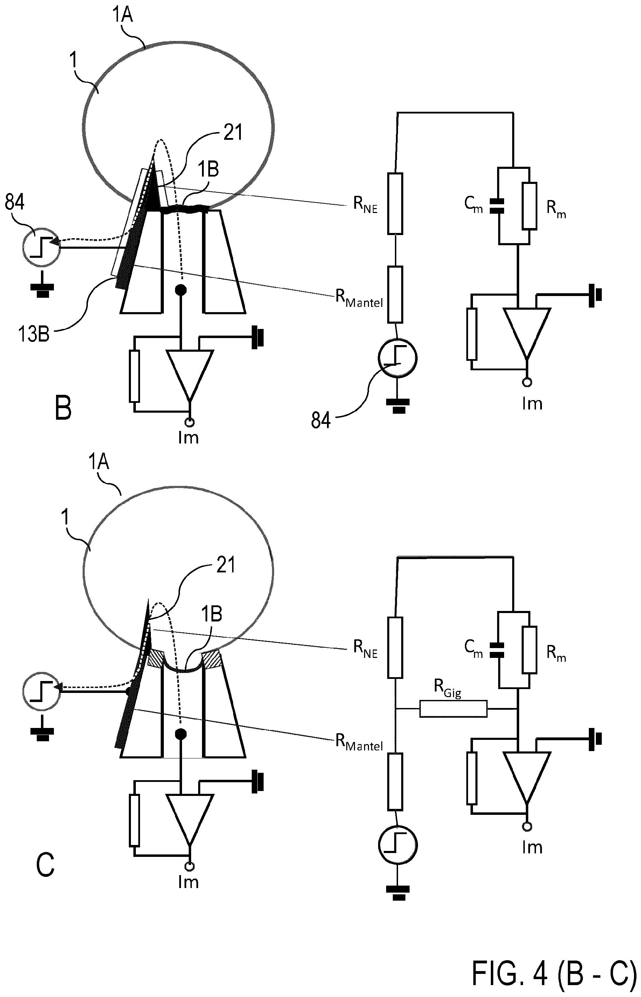

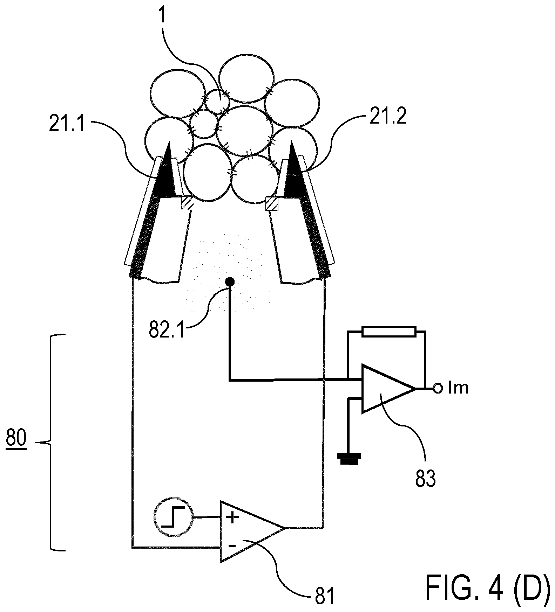

Bei der in Figur 4 gezeigten Variante wird die Substrateinrichtung 10 des elektrophysiologischen Messgeräts 100 durch den Mündungsbereich 13A einer Saugrohreinrichtung gebildet (keine Ausführungsform der Erfindung). Die Saugrohreinrichtung ist zum Beispiel eine Messpipette 13 (teilweise gezeigt), die für eine patch-clamp-Messung ausgelegt ist (patch-clamp-Pipette). Der Rand des Mündungsbereichs 13A (Pipettenspitze) am Ende der Messpipette 13 hat vorzugsweise eine Dicke d im Bereich von z. B. 1 µm bis 10 µm. Der Rand des Mündungsbereichs 13A trägt eine adhäsive Oberflächenbeschichtung zur Unterstützung der Bildung des Sealwiderstandes. Der Innendurchmesser D des Mündungsbereichs 13A ist im Bereich von z. B. 1 µm bis 200 µm gewählt, wobei die geringen Innendurchmesser (bis z.B. 30 µm) für Messungen an einzelnen Zellen und D > 10 µm für cell-attached patch-clamp-Messungen an Zellaggregaten verwendet werden. Die Elektrodeneinrichtung 20 umfasst zwei oder mehr Nanoelektroden 21 (eine der Elektroden zu Illustrationszwecken in Schnittdarstellung gezeigt), die am Umfangsrand des Mündungsbereichs 13A vorstehend angeordnet sind. Die Longitudinalrichtungen der Nanoelektroden 21 erstrecken sich parallel zu der Axialrichtung der Messpipette 13.

Verbindungsleitungen 27 verlaufen von den Nanoelektroden 21 zu Messschaltungen 81 einer Messeinrichtung 80. Die Verbindungsleitungen 27 sind außerhalb der Mündungsöffnung, zum Beispiel auf der äußeren Oberfläche der Messpipette 13, befestigt und relativ zur Umgebung elektrisch isoliert (Figuren 4A und 4B) oder frei liegend (Figur 4C). Die Messeinrichtung 80 umfasst zusätzlich eine Referenzelektrode 82, die im Inneren der Messpipette angeordnet ist, und einen Messverstärker 83, der für die Messung von Ionen-Strömen ausgelegt ist.

Bei der Ausführungsform der Erfindung umfasst die Stelleinrichtung für die Relativbewegung der Zellprobe 1 (Figur 4B, 4C und 4D) und der Nanoelektroden 21 eine Saugeinrichtung 30. Diese wird bei der Variante gemäß Figur 4 durch die Mündungsöffnung 34 der Saugrohreinrichtung (Mündung der Messpipette) und eine mit der Saugrohreinrichtung verbundene Pumpeinrichtung (Kolben der Pipette, nicht dargestellt) gebildet. Der Mündungsbereich 13A bildet die Saugöffnung, an deren Rand die Nanoelektroden 21 angeordnet sind.

Die Nanoelektroden 21 können eine Isolationsschicht 23 tragen, welche die Nanoelektroden 21 bedeckt, wobei die Elektrodenspitzen 26 unbeschichtet bleiben (Figur 4B). Die Elektrodenspitzen 26 ragen z. B. etwa 100 nm bis 5 µm aus der Isolationsschicht 23 heraus. Die Isolationsschicht 23 kann den Kontakt zur Zellmembran verbessern, was sowohl für Einzelzellen und als auch für Zellaggregate von Vorteil ist. Die Isolationsschicht 23, z. B. aus SiO₂, ist bei Mündungsöffnungen 34 mit einem Innendurchmesser z. B. im Bereich von 10 bis 100 µm und Messungen an Zellaggregaten vorgesehen, die über Nexusverbindungen verbunden sind.

Keine Isolationsschicht 23 ist beispielsweise erforderlich, wenn so genannte "cell-attached"-Messungen an Einzelzellen ausgeführt werden und sich dabei ein hochohmiger Kontakt (> 100 Megaohm) oder ein Giga-Seal am Rand des Mündungsbereichs 13A bildet (Figuren 4C und 4D). Dies wäre z. B. für einen Innendurchmesser D der Mündungsöffnung 34 im Bereich von 1 bis 30 µm, einer Dicke im Bereich von 2 bis 5 µm und dem Ansaugen einer einzelnen Zelle der Fall.

Bei Ausführung des elektrophysiologischen Messverfahrens mit dem elektrophysiologischen Messgerät 100 gemäß Figur 4 (keine Ausführungsform der Erfindung) wird in einem Gefäß (nicht dargestellt) eine Zellsuspension bereitgestellt, welche die zu untersuchenden Zellen oder Zellaggregate enthält. Die Messpipette wird in die Zellsuspension eingeführt. Bei Beaufschlagung der Messpipette mit einem Unterdruck wird eine Zelle oder ein Zellaggregat an den Mündungsbereich 13A angesaugt (cell-attached-Konfiguration), wobei die Zelle zu den Nanoelektroden 21 bewegt wird, bis diese in das Zellinnere eindringen und einen elektrischen Kontakt mit dem Zytosol in der Zelle herstellen. Zugleich wird der Membranpatch 1B gebildet (siehe Figuren 4B, 4C). Die elektrophysiologische Messung, wie zum Beispiel eine patch-clamp-Messung erfolgt in an sich bekannter Weise. An vielzelligen Proben werden Membranpotentiale an unterschiedlichen Zellen gemessen, die z. B. bei myokardialen Zellproben miteinander durch Nexuskontakte verbunden sind. Hier können mehrere Potentiale verschiedener Zellen gemessen und als Bezugsgrößen für "cell-attached" Messungen verwendet werden.

Ein besonderer Vorteil des in Figur 4 gezeigten Messgeräts besteht darin, dass durch die Positionierung der Nanoelektroden 21 am Mündungsbereich 13A keine weitere Referenzelektrode im Gefäß mit der Zellsuspension enthalten sein muss. Die Messpipette 13 ist frei handhabbar. Es ergeben sich insbesondere Vorteile beim Lösungswechsel für Testungen von Substanzen oder dergleichen. Des Weiteren kann das intrazelluläre Potential (V) direkt abgegriffen und als Bezugswert für die patch-clamp-Messung verwendet werden. In der cell-attached-Konfiguration können lonenströme durch den isolierenden Membranabschnitt und gleichzeitig die Aktionspotentiale gemessen werden, die das Zellaggregat oder die Einzelzelle generieren.

Bei der in Figur 4B dargestellten Variante (keine Ausführungsform der Erfindung) können patch-clamp-Messungen ohne das Herstellen einer Gigaseal-Isolation des Membranpatches und der Zellmembran 1A durchgeführt werden. Mit weiteren Einzelheiten zeigt Figure 4B eine schematische Darstellung des Stromflusses, ausgehend von der "virtuellen Erde" (virtual ground, I-V Konverter, schwarzer Punkt in Pipette) durch den Membranpatch der Zellmembran 1A, die Nanoelektrode 21, einen äußeren leitenden (z.B. Gold) Metallmantel 13B, der die Messpipette 13 umgibt, durch zum Spannungsgenerator 84, welcher ein Spannungsklemmsignal generiert. Im Unterschied zur herkömmlichen patch-clamp-Technik ist die Badlösung nicht mit der Messeinrichtung 80 (patch-clamp Verstärker) verbunden.

In einer abgewandelten Variante (Figur 4C, keine Ausführungsform der Erfindung) können derartige Messungen unter Bedingungen einer Gigaseal-Isolation (schraffierte Flächen an Pipettenspitze) des Membranpatches durchgeführt werden. In diesem Fall ist eine äußere Isolation der Nanoelektrode 21 mit einer Isolationsschicht 23 (z.B. SiO₂) nicht erforderlich. Der Membranpatch wird durch den Kontakt mit der Pipettenspitze von der restlichen Zellmembran 1A und Badlösung isoliert. In dieser Variante wird das Spannungsklemmsignal über die Nanoelektrode (R_{NE}) an die virtuelle Erde appliziert, wobei den Gigasealwiderstand (RGig) den Membranpatch von der Badlösung isoliert.

In einer weiteren abgewandelten Variante (Figur 4D, keine Ausführungsform der Erfindung) ist die Pipette außen mit zwei voneinander isolierten Leiterbahnen versehen, die von der Messeinrichtung 80 unmittelbar bis zur Pipettenspitze (Patch-Pore) verlaufen. Auf der Pipettenspitze sind zwei Nanoelektroden 21.1, 21.2 vorgesehen, die über die zwei Leitungsbahnen jeweils den (-) Eingang und den Ausgang eines Operationsverstärkers der Messschaltung 81 für 2 Elektroden-voltage-clamp kontaktieren. Mit der Nanoelektrode 21.1 wird dabei das Zellmembranpotential an der Zellprobe 1 in Gestalt eines Zellaggregats gemessen und über die Nanoelektrode 21.2 ein Rückkopplungsstrom zur Konstanthaltung des Membranpotentials injiziert. Die Badlösung ist dabei nicht mit einem separaten Erdpotential (ground) verbunden. Die Messeinrichtung 80 umfasst des Weiteren den Messverstärker 83 für die Messung von Ionen-Strömen, insbesondere für eine patch-clamp-Strommessung, der mit einer Referenzelektrode 82 im Inneren der Pipette verbunden ist.

Da die myokardialen Zellen des Zellaggregats durch Nexus-Kontakte miteinander verbunden sind, wird das Potential an den an die patch-Pore angesaugten Zellen konstant gehalten. Die lonenströme an dem "geklemmten" Membranpatch werden mit dem Strom-Spannungs-Konverter gemessen. Der Innendurchmesser der Pipettenspitze beträgt zwischen 10 und 200 µm.

Die Herstellung der in Figur 4 gezeigten Variante des Messgeräts (keine Ausführungsform der Erfindung) umfasst eine Mehrfachbeschichtung der Glasoberfläche der Messpipette 13 mit einer Schichtfolge, die auf der Glasoberfläche eine durch Sputtern erzeugte, strukturierte Gold-Schicht mit einer Dicke im Bereich von 100 nm bis 500 nm zur Bildung der Leiterbahnen und darauf eine SiO₂-Schicht mit einer Dicke im Bereich von 50 nm bis 500 nm umfasst. Die Nanoelektroden 21.1, 21.2 werden angeordnet, indem sie z. B. auf der Gold-Schicht an der Mündungsöffnung aufgesetzt werden.

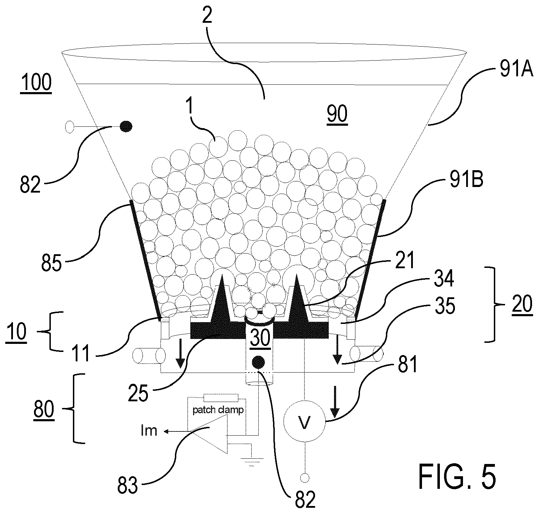

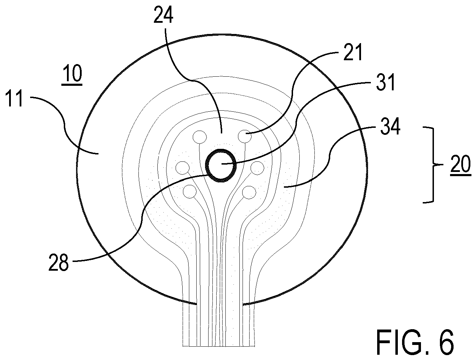

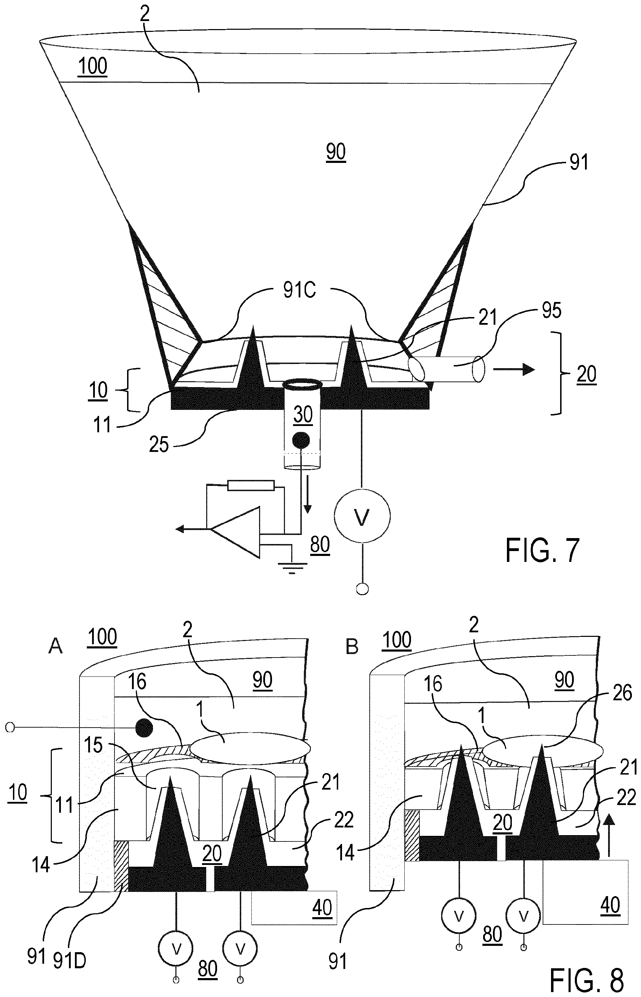

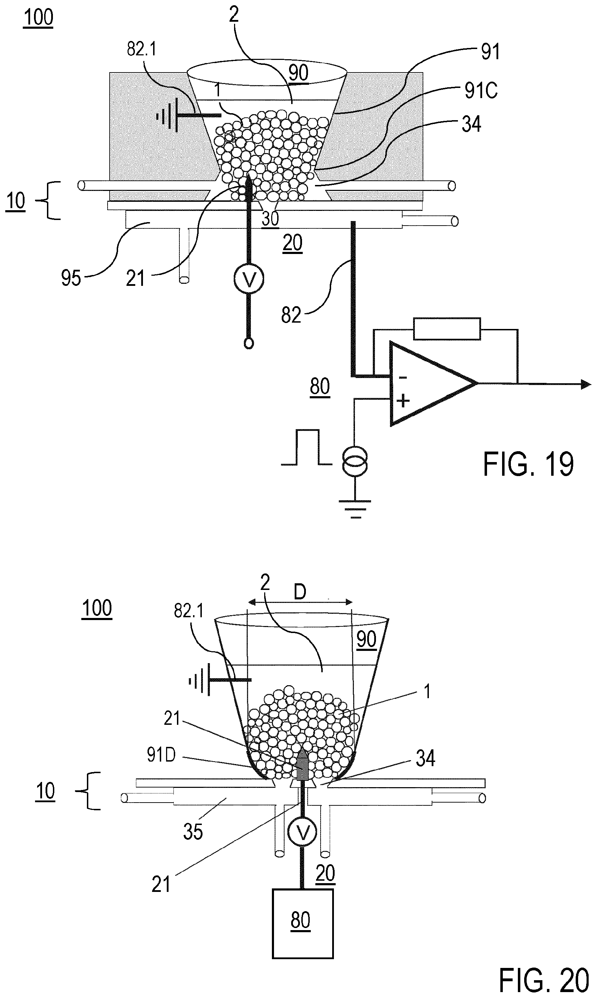

Die Figuren 5 bis 6 illustrieren weitere Abwandlungen des elektrophysiologischen Messgeräts 100 (keine Ausführungsformen der Erfindung) und Figur 7 illustriert eine Ausführungsform des elektrophysiologischen Messgeräts 100 gemäß der Erfindung), jeweils mit einer Substrateinrichtung 10, einer Elektrodeneinrichtung 20, umfassend einen Elektrodenträger 22 und Nanoelektroden 21, und einer Saugeinrichtung 30 in schematischer Schnittansicht (Figuren 5, 7) bzw. schematischer Draufsicht (Figur 6). Oberhalb der Substrateinrichtung 10 ist eine Kammer 90 mit einer sich zum Substrat 11 hin verengenden Konusform vorgesehen. Die Kammer 90 ist zur Aufnahme eines Kultivierungsmediums 2 und der Zellprobe 1 in Gestalt eines Zellaggregats eingerichtet (siehe Figur 5, keine Ausführungsform der Erfindung). Weitere Varianten dieser Ausführungsform werden unten unter Bezug auf die Figuren 19 (Ausführungsform der Erfindung) und 20 bis 21 (keine Ausführungsformen der Erfindung) beschrieben.

Die Kammerwand der Kammer 90 hat gemäß Figur 5 einen oberen, flacheren Wandabschnitt 91A und einen unteren, steileren Wandabschnitt 91B (keine Ausführungsform der Erfindung). Diese Unterteilung kann Vorteile bei einer Fixierung der Zellprobe 1 in der Kammer 90 haben. Der untere, steilere Abschnitt 91B kann mit einer inneren, elektrisch leitfähigen Beschichtung versehen sein, die eine Referenzelektrode 85 für eine elektrophysiologische Messung bildet oder für Impedanzmessungen zur Erfassung von Bewegungen der Zellaggregate dient.

Die Substrateinrichtung 10 weist ein Substrat 11 auf, das wie in Figur 1 durch den Elektrodenträger 22 mit elektrisch leitfähigen Trägerabschnitten 25 und Isolationsabschnitten 24 (siehe Figur 6, keine Ausführungsform der Erfindung) gebildet ist. Die Isolationsabschnitten 24 unterteilen den Elektrodenträger 22 in die Trägerabschnitte 25, die jeweils mit einer Nanoelektrode 21 und einer Messschaltung 81 der Messeinrichtung 80 verbunden sind. Die Nanoelektroden 21 haben eine Höhe im Bereich von z. B. 1 bis 10 µm. Die Messeinrichtung 80 umfasst die Messschaltungen 81, die jeweils mit den Nanoelektroden 21 verbunden sind, und einen Messverstärker 83, der für die Messung von Ionen-Strömen, insbesondere eine patch-clamp-Strommessung ausgelegt und mit einer Referenzelektrode 82 verbunden ist.

Die Saugeinrichtung 30 umfasst eine zentrale Saugöffnung 31 im Substrat 11, die mit einer Pumpeinrichtung (nicht dargestellt) zur Anwendung eines Unterdrucks verbunden ist. Zusätzlich ist eine Ringöffnung 34 vorgesehen, welche die Nanoelektroden 21 in der Bezugsebene des Substrats 11 umgibt. Die Ringöffnung 34 kann mit derselben Pumpeinrichtung oder, wie in Figur 5 dargestellt ist, über einen Durchflusskanal 35 unterhalb der Elektrodeneinrichtung 20 mit einer gesonderten Pumpeinrichtung verbunden sein. Die äußere Ringöffnung 34 dient dem Ansaugen der Zellprobe 1 und der Fixierung im Trichter der Kammer 90. In einer abgewandelten Variante kann die Ringöffnung 34 durch mehrere Saugöffnungen ersetzt werden, welche auf einem Kreis um die zentrale Saugöffnung 31 gleichmäßig verteilt angeordnet sind.

Die Ausführungsform der Erfindung gemäß Figur 7 ist ähnlich aufgebaut wie das elektrophysiologische Messgerät 100 gemäß Figur 5. Abweichend von diesem ist keine Ringöffnung im Substrat 11 vorgesehen. Des Weiteren bildet die Kammerwand 91 mit einem Abstand vom Substrat 11 einen sich in Umfangsrichtung und radial nach innen erstreckenden Vorsprung 91C. Der Vorsprung 91C bildet eine Unterschneidung, mit der die Fixierung der Zellprobe 1 auf dem Substrat 11 mit den Nanoelektroden 21 zusätzlich unterstützt wird. Unterhalb vom Vorsprung 91C wird ein konzentrischer Saugkanal gebildet, der mit mindestens einer Spülleitung 95 versehen ist. Über diesen Saugkanal kann die Zellprobe 1 in der Kammer angesaugt werden, was eine Fixierung der Zellprobe 1 als Alternative zur Ringöffnung 34 gemäß Figur 5 ermöglicht und außerdem eine Perfusion der Zellprobe 1 mit Flüssigkeiten, z. B. Lösungen von Testsubstanzen, ermöglicht.

Bei Anwendung des elektrophysiologischen Messgeräts 100 gemäß den Figuren 5 bis 6 (keine Ausführungsformen der Erfindung) und 7 (Ausführungsform der Erfindung) wird zunächst ein Zellaggregat, das die Zellprobe 1 bildet, in ein Kultivierungsmedium 2 in der Kammer 90 einpipettiert. Bei Beaufschlagung der Saugöffnung 31 und/oder der Ringöffnung 34 und/oder der Spülleitung 95 mit einem Unterdruck wird die Zellprobe 1 hin zum Boden der Kammer 90 auf die Nanoelektroden 21 gezogen, bis diese die Zellen der Zellprobe 1 penetrieren. Gleichzeitig wird die Zellprobe 1 bei der Variante gemäß Figur 5 vom unteren, steileren Wandabschnitt 91B der Kammerwand fixiert. Da die Zellen der Zellprobe 1 über Nexuskontakte miteinander verbunden sind, können mit der Messeinrichtung 80 elektrische Messwerte erfasst werden, welche die gesamte Zellprobe 1 repräsentieren (siehe [16]).

Mit den Messegeräten gemäß den Figuren 5 bis 6 (keine Ausführungsformen der Erfindung) und 7 (Ausführungsform der Erfindung) können vorteilhafterweise mehrere Funktionen ausgeführt werden. Mit der Saugöffnung 31 im Zentrum und der optional vorgesehenen konzentrischen Ringöffnung 34 oder dem konzentrischen Saugkanal im unteren Trichterabschnitt wird die Zellprobe angesaugt, so dass die Penetration der Membran bewirkt wird. Des Weiteren umgibt die Saugöffnung 31 vorzugsweise ein Dichtungsring-Abschnitt 28 (sealing-Ring), der wie bei herkömmlichen patch-clamp-Messungen einen elektrischen Widerstand bildet. Der Dichtungsring-Abschnitt 28 wird durch die Oberfläche eines Isolationsabschnitts 24 gebildet.

Bei einem weiteren elektrophysiologischen Messgerät wird die Stelleinrichtung durch eine Elektroden-Antriebseinrichtung 40 gebildet, wie schematisch beispielhaft in den Figuren 8 bis 11 gezeigt ist (keine Ausführungsformen der Erfindung). Die Elektroden-Antriebseinrichtung 40 umfasst einen mechanischen Antrieb, der auf die Elektrodeneinrichtung 20, insbesondere den Elektrodenträger 22 und/oder die Nanoelektroden 21 wirkt. Bei den dargestellten Varianten umfasst die Elektroden-Antriebseinrichtung 40 einen elektrisch stimulierten Piezo-Aktor aus einem piezoelektrischen Material, wie z. B. piezoelektrische Keramiken oder piezoelektrische Kristalle. Alternativ können andere Materialien verwendet werden, die in Reaktion auf eine andere, z. B. optische, äußere Stimulation ihre geometrischen Eigenschaften verändern.

Der Elektrodenträger 22 mit den Nanoelektroden 21 ist bei diesen Varianten (keine Ausführungsformen der Erfindung) relativ zur Kammerwand 91 der Kammer 90 beweglich angeordnet, wobei zwischen der Elektrodeneinrichtung 20 und der Kammerwand 91 vorzugsweise eine Dichtung 91D vorgesehen ist, die eine gleitende Bewegung des Elektrodenträgers 22 erlaubt und zugleich flüssigkeitsdicht ist.

Gemäß einer ersten Variante des Messgeräts (siehe Figur 8, 10 und 11, keine Ausführungsformen der Erfindung) umfasst die Substrateinrichtung 10 ein Substrat 11, das einen Substratkörper 14 und/oder eine Porenmembran 16 aufweist, die jeweils durchgehende Elektrodenöffnungen 15 enthalten. Der Elektrodenträger 22 der Elektrodeneinrichtung 20 ist an der Unterseite der Substrateinrichtung 10 angeordnet. Nanoelektroden 21 der Elektrodeneinrichtung 20 ragen in die Elektrodenöffnungen 15 hin zur Kammer 90. Bei Betätigung der Elektroden-Antriebseinrichtung 40 werden die Nanoelektroden 21 durch die Elektrodenöffnungen 15 vorgeschoben.

Bei der Variante gemäß Figur 8 (keine Ausführungsform der Erfindung) ist der Substratkörper 14 aus einem komprimierbaren Material, wie zum Beispiel PVC mit einer Dicke von 100 nm bis 100 µm hergestellt. Auf der oberen, von der Elektrodeneinrichtung 20 abgewandten Seite des Substratkörpers 14 ist die Porenmembran 16 angeordnet. Die Porenmembran 16 ist aus einem unelastischen Material, wie zum Beispiel PDMS oder PVC mit einer Dicke von 100 bis 500 µm hergestellt und innen an der Kammerwand 91 befestigt. Aus Klarheitsgründen ist in Figur 8 die Porenmembran 16 mit einem Abstand vom Substratkörper 14 gezeigt. In der Realität berührt die Porenmembran 16, die sich parallel zur Bezugsebene des Substrats 11 mit dem Substratkörper 14 erstreckt, die Oberseite des Substratkörpers 14. Die Porenmembran 16 stellt eine feste Auflage für die Zellprobe 1 dar (Figur 8A).

Bei Betätigung der Elektroden-Antriebseinrichtung 40 drückt die Elektrodeneinrichtung 20 gegen den Substratkörper 14, wobei dieser zwischen der Elektrodeneinrichtung 20 und der Porenmembran 16 komprimiert wird, während die Nanoelektroden 21 durch die Elektrodenöffnungen 15 vorgeschoben werden, bis die Elektrodenspitzen 26 in die Zellprobe 1 eindringen (Figur 8B). In diesem Zustand erfolgt die Messung der gewünschten elektrischen Größen der Zellprobe 1 mit der Messeinrichtung 80.

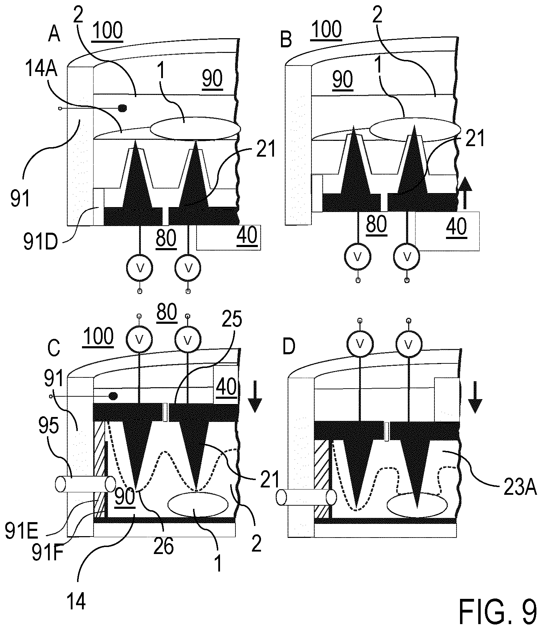

Gemäß einer alternativen Variante ist die mindestens eine Elektrodenöffnung 15 der Substrateinrichtung 10 mit einer Deckelmembran 14A verschlossen und die Elektroden-Antriebseinrichtung 40 für die Bewegung der Nanoelektroden 21 durch die Deckelmembran 14A in die Zellprobe 1 eingerichtet (Figuren 9A und 9B, keine Ausführungsformen der Erfindung). Wie in Figur 8 ist der Elektrodenträger 22 mit den Nanoelektroden 21 relativ zur Kammerwand 91 der Kammer 90 beweglich angeordnet, wobei die flüssigkeitsdichte Dichtung 91D die gleitende Bewegung des Elektrodenträgers 22 erlaubt. Die Deckelmembran 14A bildet die Substratoberfläche zur Aufnahme der Zellprobe 1, und sie ist wie die Porenmembran 16 (Figur 8) aus einem unelastischen Material hergestellt und innen an der Kammerwand 91 befestigt. Die Deckelmembran 14A wird durch eine Trenneinrichtung bereitgestellt, welche den Raum zur Aufnahme der Zellprobe 1 (Inneres der Kammer 90) von den Nanoelektroden 21 trennt, solange die Elektroden-Antriebseinrichtung 40 nicht betätigt und die Nanoelektroden 21 nicht vorgeschoben werden.

Gemäß einer weiteren alternativen Variante der Erfindung umfasst das Substrat 11 der Substrateinrichtung 10 einen Substratkörper 14 mit einer geschlossenen Oberfläche, wobei in diesem Fall Nanoelektroden 21 der Elektrodeneinrichtung 20 mit einem Abstand vom Substratkörper 14 angeordnet sind und von der Kammer 90 her zum Substratkörper 14 ragen (top-down-Konfiguration, siehe Figuren 9C und 9D, keine Ausführungsformen der Erfindung). Die Elektrodeneinrichtung 20 ist mit der Kammerwand 91 verbunden und senkrecht zur Bezugsebene des Substrats 11 verschiebbar. Bei Betätigung der Elektroden-Antriebseinrichtung 40 werden die Nanoelektroden 21 zum Substrat 11 vorgeschoben, bis die Elektrodenspitzen 26 in die Zellprobe 1 eindringen, so dass die Messung der gewünschten elektrischen Größen der Zellprobe 1 mit der Messeinrichtung 80 erfolgen kann.

Bei dem Aufbau gemäß den Figuren 9C und 9D (keine Ausführungsformen der Erfindung) kann der Substratkörper 14 eine durchsichtige Platte, z. B. aus Kunststoff oder Glas umfassen, so dass eine visuelle und/oder optisch-mikroskopische Beobachtung der Zellprobe 1 durch den Substratkörper 14 ermöglicht wird.

Optional ist der Substratkörper 14 mit einem zellverträglichen Material, wie z. B. Magel, Poly-L-Lysin, Gelatine oder Fibronektin beschichtet. Eine ebenfalls optional vorgesehene seitliche Spülleitung 95 (Perfusionskanal) dient der Zuführung von Zellen. Des Weiteren erlaubt die Spülleitung 95 die Erzeugung eines Unterdrucks, unter dessen Wirkung die Vorschub-Bewegung der Elektrodeneinrichtung 20 unterstützt wird.

Zwischen dem Elektrodenträger 25 und dem Substratkörper 14 ist ein Abstandhalter 91E (elastisches Spacer-Element, schraffiert gezeigt), z.B. aus PDMS oder Gummi, vorgesehen, der die Kammer abdichtet und zugleich mit mindestens einem Anschlagelement 91F ausgestattet ist (keine Ausführungsform der Erfindung). Der Abstandhalter 91E kann am Rand der Kammer 90 (wie dargestellt) und optional zusätzlich im Volumen der Kammer jeweils die Nanoelektroden 21 umgebend vorgesehen sein. Abstandhalter 91E können somit nicht nur am Rand der Kammer 90, sondern auch zwischen den Nanoelektroden 21 und dem Substratkörper 14 platziert sein. Es wird eine gleichzeitige Bewegung der Nanoelektroden 21 in mehrere Zellen auf einer Substratoberfläche ermöglicht, ohne dass der Elektrodenträger 25 durch die Bewegung verbogen wird. Mit dem mindestens einen Anschlagelement 91F (Begrenzungselement) kann die Bewegung der Nanoelektroden 21 eingeschränkt werden, was eine Einstellung einer definierten Eindringtiefe der Nanoelektroden 21 in das Zytosol ermöglicht.

Bei der Variante gemäß den Figuren 9C und 9D (keine Ausführungsformen der Erfindung) kann die feste SiO₂-Beschichtung der Nanoelektroden 21 entfallen. Stattdessen ist vorzugsweise eine Beschichtung 23A der Nanoelektroden 21 mit einer dünnen Schicht (z. B. 10 nm bis 100 nm oder mehr, z. B. bis 1 µm) eines viskösen Materials, beispielsweise Vaseline, Gemischen aus Silikonöl und Parafilm, Polydimethylsiloxan (PDMS), Gelatine jeweils mit Beimischungen von extrazellulärer Matrix (Magel, BioCoat, Fibronektin, Poly-L-Lysin oder Aminosäuren und Nährstoffen, vorgesehen. Bei Einführung der Nanoelektroden 21 in die Zellprobe 1 wird die Beschichtung 23A von den Nanoelektroden 21 partiell abgestreift, so dass der Kontakt mit dem Zytosol hergestellt wird (siehe Figur 9D, keine Ausführungsformen der Erfindung). Die Beschichtung 23A der Nanoelektroden 21 mit dem viskösen Material kann auch bei den anderen Ausführungsformen der Erfindung vorgesehen sein.

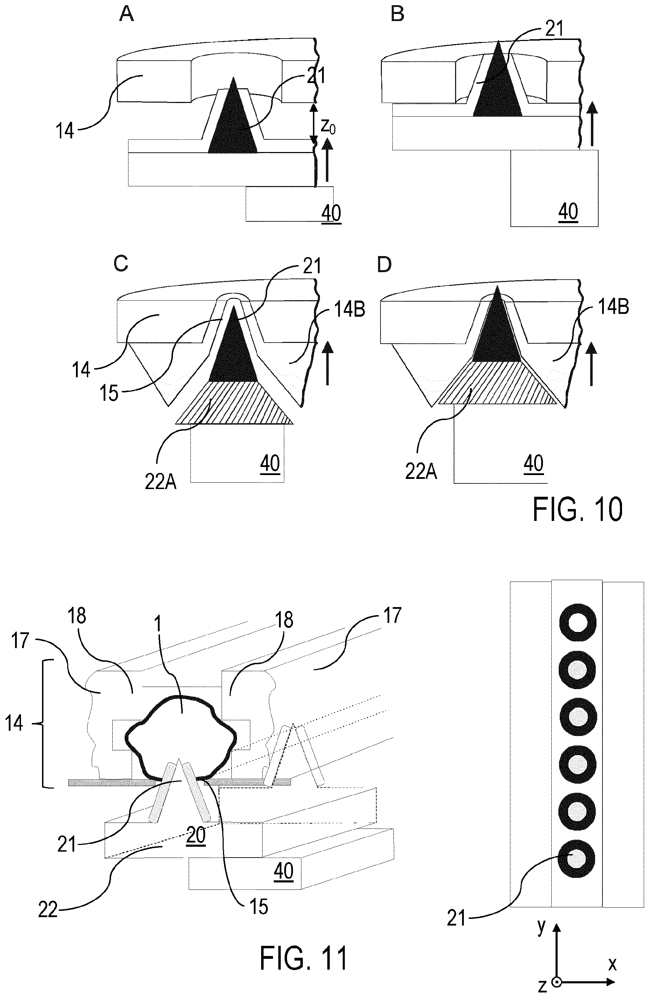

Gemäß einer weiteren alternativen Variante umfasst das Substrat der Substrateinrichtung 10 einen Substratkörper 14 mit Elektrodenöffnungen 15 aus einem nicht-komprimierbaren Material (Figuren 10, 11, keine Ausführungsformen der Erfindung). In diesem Fall ist die Elektrodeneinrichtung 20 wie in Figur 8 unterhalb des Substratkörpers 14 angeordnet, wobei die Nanoelektroden 21 in die Elektrodenöffnungen 15 ragen. Abweichend von Figur 8 ist die Elektrodeneinrichtung 20 mit einem Abstand z₀ vom Substratkörper 14 angeordnet. Der Abstand z₀ ist so gewählt, dass die Nanoelektroden 21 um einen ausreichenden Vorschubweg durch die Elektrodenöffnungen 15 in die Zellprobe 1 bewegt werden können (Figuren 10A/B). Der Abstand z₀ beträgt z. B. 100 nm bis 3 mm. Die Unterseite des Substratkörpers 14 bildet einen Anschlag für die Elektrodeneinrichtung, so dass eine definierte Eindringtiefe der Nanoelektroden 21 in der Zellprobe einstellbar ist. Zusätzlich können der Substratkörper oder das Zellarray mit flexiblen Abstandhaltern (Spacer-Elemente) und Anschlagelementen (siehe Figuren 9C, 9D, keine Ausführungsformen der Erfindung) ausgestattet sein.

Zusätzlich kann der Substratkörper 14 an seiner Unterseite mit einem Führungskonus 14B ausgestattet sein, der mit passend geformten konischen Abschnitten 22A des Elektrodenträgers 22 zusammenwirkt (Figur 10C, keine Ausführungsform der Erfindung). Vorteilhafterweise wird damit die zentrierte Führung der Nanoelektroden 21 durch die Elektrodenöffnungen 15 unterstützt. Der Führungskonus 14B kann aus einem flexiblen Material bestehen (z.B. PDMS), welches bei Bewegung der Nanoelektrode eine abdichtende und isolierende Wirkung hat. In diesem Fall kann wahlweise auf eine isolierende Beschichtung der Nanoelektrode verzichtet werden (Figur 10D, keine Ausführungsform der Erfindung).

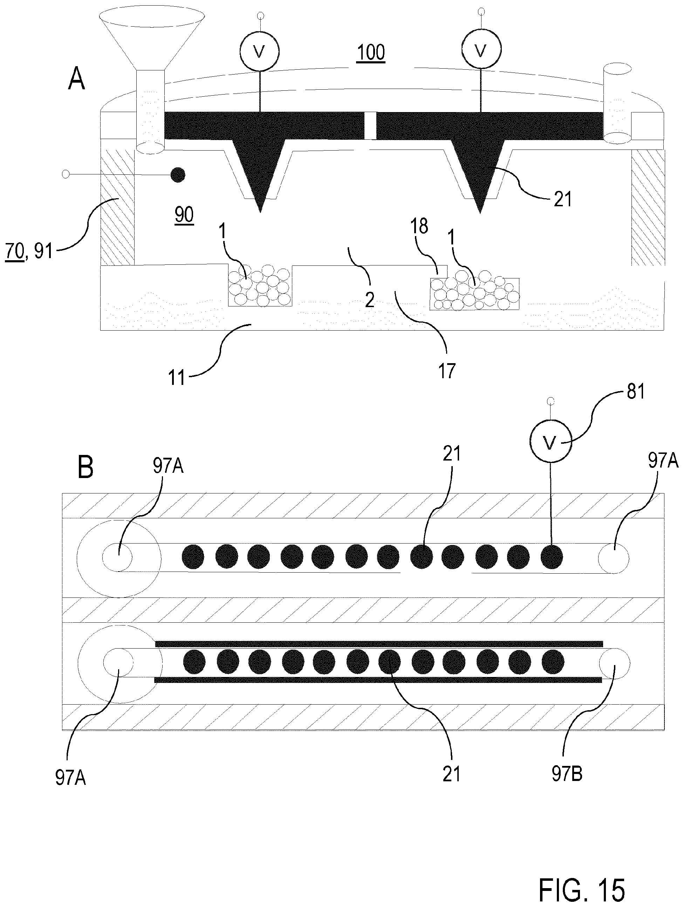

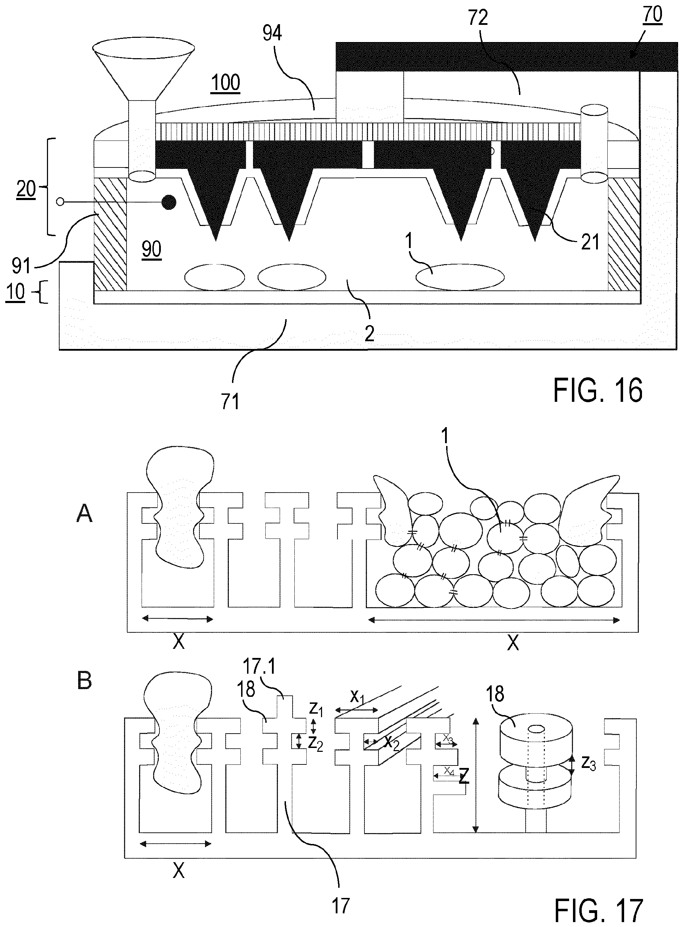

Figur 11 (keine Ausführungsform der Erfindung) zeigt eine strukturierte Oberfläche des Substratkörpers 14 mit Substratvorsprüngen 17, die lateral vorstehende Ankerelemente 18 aufweisen. Die Substratvorsprünge 17 mit den Ankerelementen 18 fixieren die Zellprobe 1 während des Einstechens der Nanoelektrode 21, wenn diese mit der Elektroden-Antriebseinrichtung 40 durch die Elektrodenöffnung 15 vorgeschoben wird. Die Substratvorsprünge 17 haben eine langgestreckte Wandform, so dass zwischen den Substratvorsprüngen 17 eine Nut (Rinne) zur Aufnahme der Zellprobe 1 gebildet wird. Die Zellprobe 1 kann z. B. eine in Längsrichtung der Nut wachsende Faser bilden, die durch Ankerelemente in der Rinne fixiert sind. Die obere Fläche von 17 ist mit Nanostrukturen versehen, die eine Zellanhaftung verhindern, die Nut wahlweise mit adhäsionsfördernden Beschichtungen versehen. Die Nanoelektroden 21 und die Elektrodenöffnungen 15 sind z. B. als Reihe angeordnet, wie mit der Draufsicht im rechten Teil von Figur 11 gezeigt ist. Die Umfangsränder der Elektrodenöffnungen 15 bilden jeweils Dichtflächen zur Auflage der Zellmembran der Zellprobe während der Messung. Weitere Einzelheiten und Funktionen der strukturierten Oberfläche sind unten unter Bezug auf die Figuren 15 und 17 (keine Ausführungsformen der Erfindung) beschrieben.

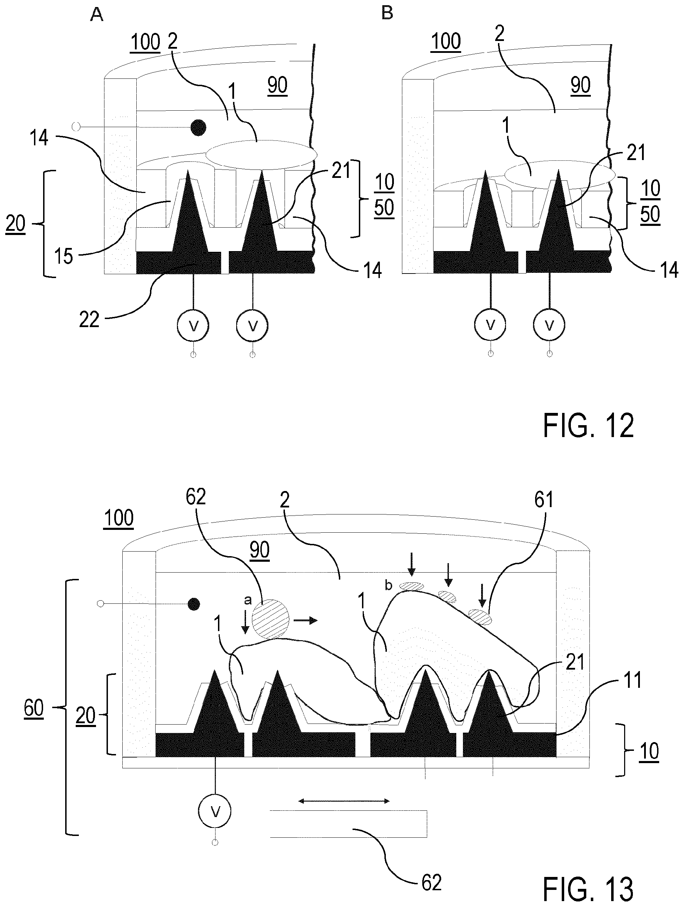

Bei einem weiteren elektrophysiologischen Messgerät (keine Ausführungsform der Erfindung) wird die Stelleinrichtung durch eine Substrat-Antriebseinrichtung 50 gebildet, wie schematisch beispielhaft in Figur 12 gezeigt ist. Die Substrat-Antriebseinrichtung 50 umfasst einen mechanischen Antrieb, der auf die Substrateinrichtung 10, insbesondere den Substratkörper 14 mit den Elektrodenöffnungen 15 wirkt. Die Elektrodeneinrichtung 20 mit den Nanoelektroden 21 und dem Elektrodenträger 22. die z. B. wie in Figur 1 gebildet ist, ist ortsfest am Boden der Kammer 90 angeordnet. Die Substrat-Antriebseinrichtung 50 wird durch den Substratkörper 14 selbst gebildet, der z. B. aus einem elektrisch, optisch, magnetisch oder mit Ultraschall stimulierbaren Material hergestellt ist. In Reaktion auf eine äußere Stimulation, z. B. eine Änderung einer elektrischen Spannung, die an dem Substratkörper 14 anliegt, erfolgt eine Kontraktion des Substratkörpers 14.

Die Dicke des Substratkörpers 14 verringert sich (Figuren 12A/12B), bis die Nanoelektroden 21 über die obere Oberfläche des Substratkörpers 14 vorragen und in die Zellprobe 1 eindringen. Der Substratkörper 14 weist auf seiner Oberseite vorzugsweise eine zellverträgliche Beschichtung, z. B. aus SiO₂, auf, welche die Substratoberfläche zur Aufnahme der Zellprobe bildet.

Bei einem weiteren elektrophysiologischen Messgerät (keine Ausführungsform der Erfindung) wird die Stelleinrichtung durch eine Magnet-Antriebseinrichtung 60 gebildet, wie schematisch beispielhaft in Figur 13 gezeigt ist. Die Magnet-Antriebseinrichtung 60 umfasst magnetische Partikel 61, die mit der Zellprobe 1 gekoppelt sind, und einen Hauptmagneten 62, der unterhalb der Elektrodeneinrichtung 20 angeordnet ist. Die magnetischen Partikel 61 sind z. B. glasummantelte Mikro-Eisenkugeln oder magnetische Nanopartikel. Der Hauptmagnet 62 ist ein Permanentmagnet oder ein schaltbarer Elektromagnet. Der Hauptmagnet 62 ist dafür vorgesehen, bei magnetischer Wechselwirkung mit den magnetischen Partikeln 61 die Zellprobe 1 zu den Nanoelektroden 21 zu ziehen, bis sie in die Zellen der Zellprobe 1 eindringen.

Die Zellprobe 1 kann in einer Richtung senkrecht zur Bezugsebene des Substrats 11 und optional zusätzlich seitlich entlang der Bezugsebene des Substrats 11 bewegt werden. Für die seitliche Bewegung kann eine Verschiebung des Hauptmagneten 62 parallel zu der Bezugsebene des Substrats 11 vorgesehen sein. Einzelheiten der Bewegung der Zellprobe 1 können z. B. realisiert werden, wie in [17] beschrieben ist.

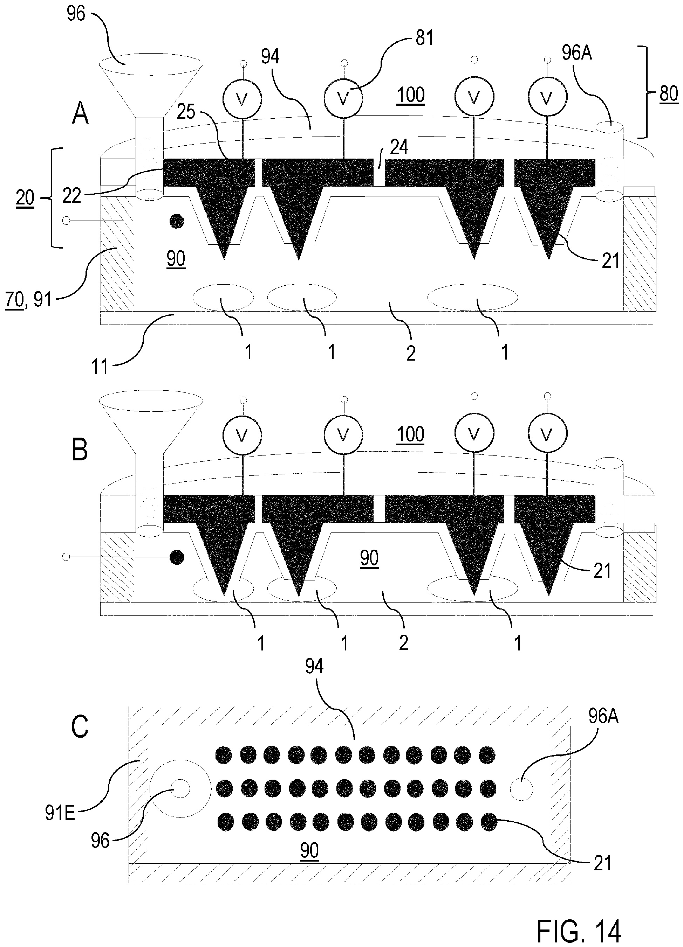

Bei einem weiteren elektrophysiologischen Messgerät (keine Ausführungsform der Erfindung) wird die Stelleinrichtung durch eine Kammer-Antriebseinrichtung 70 gebildet, die an einer mit der Substrateinrichtung 10 verbundenen Kammerwand 91 vorgesehen und für eine Bewegung der Nanoelektroden 21 zu der Zellprobe 1 eingerichtet ist (siehe Figuren 14 bis 16). In den illustrierten Varianten wird die Kammer-Antriebseinrichtung 70 durch die Kammerwand 91 selbst gebildet. Alternativ kann die Kammer-Antriebseinrichtung 70 eine gesonderte Komponente sein, die mit der Kammerwand 91, z. B. aus einem elastisch deformierbaren Material, oder einem anderen Teil der Kammer 90 verbunden ist und bei Betätigung die Kammerwand 91 deformiert (siehe z. B. Figur 16).

Diese Variante wird als top-down-Konfiguration realisiert, bei der die Elektrodeneinrichtung 20 mit den Nanoelektroden 21 mit einem Abstand über dem ortsfesten Substrat 11 der Substrateinrichtung 10 angeordnet ist. In der top-down-Konfiguration wird durch die Kammerwand 91 eine Trenneinrichtung bereitgestellt, welche die Zellprobe 1 im Inneren der Kammer 90 von den Nanoelektroden 21 trennt, solange die Kammer-Antriebseinrichtung 70 nicht betätigt und die Nanoelektroden 21 nicht vorgeschoben werden (Figuren 14A, 15A und 16).

Gemäß Figur 14A (keine Ausführungsform der Erfindung) wird die Kammer 90 des elektrophysiologischen Messgeräts 100 nach unten durch das Substrat 11, z. B. aus Glas, seitlich durch die Kammerwand 91 und nach oben durch ein Deckelteil 94, z. B. aus Glas, begrenzt, in das die Elektrodeneinrichtung 20 und ein Zufuhrtrichter 96 integriert sind. Die Elektrodeneinrichtung 20 umfasst einen Elektrodenträger 22, der durch Isolationsabschnitte 24 in Trägerabschnitte 25 jeweils mit einer Nanoelektrode 21 unterteilt ist, wie oben beschrieben ist. Jede Nanoelektrode 21 ist mit einer Messschaltung 81 der Messeinrichtung 80 verbunden. Die Kammerwand 91 besteht aus einem piezoelektrischen Material, das sich bei Änderung einer elektrischen Spannung kontrahiert. Alternativ kann die Kammerwand aus einem elastischen Material bestehen, welches durch eine Kammer-Antriebseinrichtung komprimiert werden kann.

Zur Ausführung einer elektrophysiologischen Messung wird das Messgerät 100 über den Zufuhrtrichter 96 mit einer Zellsuspension, umfassend die Zellprobe 1 und ein Kultivierungsmedium 2 gefüllt. Die Innenmaße und die innere Oberfläche des Zufuhrtrichters 96 und der Kammer 90 können so gebildet sein, dass die Zufuhr der Zellsuspension durch Kapillarkräfte unterstützt wird. Vorteilhafterweise haftet die Zellprobe 1 an der Oberfläche des Substrats 11 an, bevor sich die Nanoelektroden 21 bewegen. Die Oberfläche des Substrats 11 kann, wie unter Bezug auf Figur 17 beschrieben ist, strukturiert sein, um die Anhaftung zu fördern (Nanostrukturierung als Adhäsionshilfe). Nach der Zufuhr der Zellsuspension haben die Nanoelektroden 21 einen Abstand von den Zellen der Zellprobe 1 (Figur 14A).

Bei elektrische Stimulation der Kammer-Antriebseinrichtung 70, d. h. der Kammerwand 91, wird die Elektrodeneinrichtung 20 abgesenkt, bis die Nanoelektroden 21 die Zellen der Zellprobe 1 penetrieren (Figur 14B). In diesem Zustand erfolgt die Erfassung mindestens eines elektrischen Messwerts an der Zellprobe 1.

Die Kammer 90 des elektrophysiologischen Messgeräts 100 kann ein geschlossenes Gefäß mit einem zylinderförmigen Innenraum sein (Figuren 14A und 14B, keine Ausführungsform der Erfindung). Alternativ kann die Kammer 90 einen quaderförmigen Innenraum haben und insbesondere als Durchfluss-System (Kanal) gebildet sein, wie in der Draufsicht in Figur 14C gezeigt ist. Die Nanoelektroden 21 im Deckelteil 94 können z. B. ein Elektroden-Array, insbesondere für Messungen an Zellrasen (Monolayer), bilden. Figur 14C illustriert des Weiteren, dass die Kammer 90 seitlich von einem Abstandhalter 91E abgedichtet sein kann (keine Ausführungsform der Erfindung).

Die Figuren 15A und 15B zeigen eine Abwandlung des elektrophysiologischen Messgeräts 100 gemäß Figur 14, wobei das Substrat 11 eine strukturierte Oberfläche mit Substratvorsprüngen 17 und seitlichen Ankerelementen 18 hat (keine Ausführungsformen der Erfindung). Die Substratvorsprünge 17 bilden gerade Nuten am Boden der Kammer 90, in denen die Zellprobe 1 adhärent positioniert wird. Die Nuten haben z. B. eine Breite von 10 µm bis 300 µm und eine Tiefe von 3 µm bis 300 µm. Die seitlichen Ankerelementen 18 haben z. B. eine Ausdehnung parallel zur Bezugsebene des Substrats von 100 nm bis 100 µm. Das adhärente Wachstum ausschließlich oder überwiegend in den Nuten kann durch eine adhäsionsfördernde Beschichtung in den Nuten unterstützt werden. Während Zellen ohne die Ankerelemente 18 möglicherweise nur oberflächlich in den Nuten haften und sich insbesondere bei Bildung von kontrahierenden Herzmuskelfasern von dem Substrat 11 lösen (siehe [14], [15]), bieten die Ankerelemente 18 eine zusätzliche Fixierung der Zellen.

Figur 15B zeigt eine Draufsicht auf eine Variante des elektrophysiologischen Messgeräts 100, bei dem die Nanoelektroden 21 zwei gerade Reihe bilden, die entlang der Ausrichtung der Nuten verlaufen, welche durch die Substratvorsprünge 17 gebildet werden (keine Ausführungsform der Erfindung). An jeder der Nanoelektroden 21 ist eine Messschaltung 81 vorgesehen (nur an einer der Nanoelektroden 21 illustriert). An den entgegengesetzten Enden jeder Reihe ist jeweils eine Stimulationselektrode 97A, 97B zur Reizung und Erregungsausbreitung in eine Richtung entlang der Reihe vorgesehen.

Bei der Variante gemäß Figur 15 sind seitliche Zugänge der Kammer offen. Die Kammer wird bei dieser Variante z. B. in einem Zellkulturgefäß angeordnet und anschließend in einer seitlich abdichtenden Perfusionseinrichtung platziert, damit die Kammer entlang der Vertiefungen im Boden (Nuten mit und ohne Ankerelemente) perfundiert werden kann. Die offene Variante der Figur 15 gewährleistet den Zugang größerer Volumina von Kulturmedium in einem Zellkulturgefäß durch Diffusion zu den Zellen. Das Volumen ist in der sehr flachen geschlossenen Version Fig. 14C durch die geringe Höhe begrenzt.

Die Varianten insbesondere der Figuren 15A und 15B (keine Ausführungsformen der Erfindung) haben des Weiteren den besonderen Vorteil, dass intrazelluläre Messungen der Ausbreitung eines Aktionspotentials über eine Zellkultur, z. B. eine Faserstruktur, ermöglicht wird. Dies war mit herkömmlichen Techniken nur mit extrazellulären Elektroden bekannt. Die Ausbreitung des Aktionspotentials kann insbesondere in Abhängigkeit von der Einwirkung einer Perfusionsflüssigkeit gemessen werden. Die Perfusionsflüssigkeit kann z. B. biologische wirksame Substanzen, insbesondere Arzneistoffe, enthalten, deren Wirkung auf die Zellprobe 1 durch die elektrophysiologische Messung erfasst werden soll.

In Figur 16 ist eine abgewandelte Variante der Kammer-Antriebseinrichtung 70 gezeigt, die eine Halterung 71 und einen Hebel-Aktor 72 umfasst (keine Ausführungsform der Erfindung). In der Halterung 71 ist die Kammer 90 mit der Substrateinrichtung 10 und der Elektrodeneinrichtung 20 fixiert. Das Deckelteil 94 mit der Elektrodeneinrichtung 20 ist relativ zu der Kammerwand 91 beweglich angeordnet. Der Hebel-Aktor 72 wirkt auf das Deckelteil 94. Die Kammerwand 91 ist aus einem elastischen (komprimierbaren) Material hergestellt. Bei Betätigung der Kammer-Antriebseinrichtung 70 wird mit dem Hebel-Aktor 72 das Deckelteil 94 nach unten verschoben, so dass alle Nanoelektroden 21 gleichzeitig zu der Zellprobe 1 bewegt werden und in die Zellen eindringen. Der Hebel-Aktor 72 umfasst z. B. ein piezoelektrisches Material oder alternativ eine Kombination von zwei Materialien mit verschiedenen thermischen Ausdehnungskoeffizienten, z. B. ein Bi-Metall-Komposit. Das Bi-Metall-Komposit hat den Vorteil eines größeren Vorschubweges (z. B. bis 100 µm oder darüber), im Vergleich zu einem piezoelektrische Material (z. B. bis 2 bis 5 µm).

Als Abwandlung der Variante in Figur 16 kann der Aktor 72 Teil des Elektrodenträgers 22 sein (keine Ausführungsform der Erfindung).

Zur Fixierung einer Zellprobe 1, insbesondere von Einzelzellen und/oder Zellaggregaten, auf dem Substrat eines elektrophysiologischen Messgeräts, z. B. auf dem Boden in einem Perfusionsspalt, können Substratvorsprünge 17 mit seitlichen Ankerelementen 18 vorgesehen sein, wie in den Figuren 17A und 17B gezeigt ist (keine Ausführungsformen der Erfindung). Die Substratvorsprünge 17 mit seitlichen Ankerelementen 18 können bei allen hier beschriebenen Ausführungsformen der Erfindung vorgesehen sein, die mit einer Stelleinrichtung ausgestattet sind. Alternativ können die Substratvorsprünge 17 mit seitlichen Ankerelementen 18 bei Messgeräten vorgesehen sein, die keine Stelleinrichtung aufweisen und bei denen somit keine gezielte Relativbewegung von Zellprobe und Nanoelektroden vorgesehen ist (keine Ausführungsformen der Erfindung). Des Weiteren können die Substratvorsprünge 17 mit seitlichen Ankerelementen 18 bei Substraten, z. B. in Gestalt von Deckgläsern mit einer strukturierten Oberfläche, vorgesehen sein, bei denen keine Nanoelektroden vorgesehen sind (keine Ausführungsformen der Erfindung). Die Herstellung der Substratvorsprünge mit den Ankerelementen erfolgt z. B. mit der Nanoimprint-Lithographie-Technologie.

Die verbesserte Fixierung der Zellprobe ist von Vorteil, da Zellen mit geringer Haftung beispielsweise aus der Kammer gespült werden könnten, wenn in einer Perfusionsflüssigkeit durch den Lösungsfluss Scherkräfte auftreten.

Die Figuren 17A und 17B (keine Ausführungsformen der Erfindung) zeigen Substratvorsprünge 17 in Säulen- oder Wandform, die an ihren oberen Enden jeweils seitliche Ankerelemente 18 übereinander aufweisen (so genannte Doppel-oder Dreifach-T-Profile). Alternativ können die Substratvorsprünge 17 z. B. einfache T-Profile bilden oder übereinander mehr als drei seitliche Ankerelemente 18 aufweisen. Die Doppel-T-Profile zeichnen sich durch einen Abstand X der Substratvorsprünge 17 aus, der für die Fixierung von Einzelzellen z. B. im Bereich von 300 nm bis 10 µm und für die Fixierung von Zellaggregaten und Fasern z. B. im Bereich von 30 µm bis 1 mm gewählt ist. Des Weiteren zeichnen sich die Doppel-T-Profile durch eine Höhe Z der Substratvorsprünge 17 im Bereich von 500 nm bis 1 mm eine Breite der Ankerelemente x₁ im Bereich von 100 nm bis 1 mm, eine Unterschneidungstiefe der Ankerelemente x₂ im Bereich von 100 nm bis 100 µm, eine Dicke der Ankerelemente z₁ im Bereich von 100 nm bis 500nm und einen Abstand der Ankerelemente z₂ im Bereich von 50 nm bis 10 µm aus.

Bei Mehrfach-T-Profilen können die Ankerelemente parallel zur Bezugsebene des Substrats verschiedene, z. B. zum Substrat hin steigende, Ausdehnungen haben (siehe Figur 17B). Beispielsweise ist die Dimension X3 zwischen 2% und 50% größer als X2, bei einem weiteren Ankerelement könnte eine Dimension X4 um 5-50% größer als X3 sein.

Die Oberseite der T-Strukturen weist eine geringere Haftung für Zellen auf als die übrigen Oberflächen, was eine bevorzugte Anhaftung und ein Wachstum der Zellen am Boden entlang der Vertiefung (Rille) bewirkt. Eine Noppe 17.1 z.B. zur Verhinderung der Anhaftung ist beispielhaft in Figur 17B gezeigt (keine Ausführungsform der Erfindung).

Die Arrays für Fasern in T-Strukturen ermöglichen die Messung von Aktionspotentialen (APs) in verschiedenen Stellen entlang und damit die Bestimmung der Ausbreitungsgeschwindigkeit oder auch des interzellulären Widerstandes. Vorteilhafterweise erfolgt die Messung an einer mit den Nuten ausgerichteten Struktur, was durch die vereinfachte Geometrie - im Vergleich zum ungerichteten Wachstum in einem Monolayer - diese Messungen erleichtert.

Alternativ oder zusätzlich sind verschiedene geometrische Ausrichtungen, Verjüngungen der Rillen, ein kreisförmiger Verlauf (2 Fasern treffen sich) der zwischen den Substratvorsprüngen gebildeten Nuten realisierbar.

Die Substratvorsprünge 17 können langgestreckte Nano-Nuten bilden, die z. B. Haftungselemente für Filopodia von einzelnen Zellen oder Zellaggregaten bilden. Zwischen den Substratvorsprüngen 17 können im Substrat Öffnungen vorgesehen sein, in denen Elektroden verfahrbar sind (siehe z. B. Figur 11, keine Ausführungsform der Erfindung). Alternativ können Vertiefungen zwischen Substratvorsprüngen 17, z. B. mit T-Strukturen, neben einer geraden Ausrichtung (Wachstum einfacher Längsfaserrichtungen) andere geometrische Formen haben, wie z.B. kreuzförmig verlaufen, kreisförmig verlaufen und/oder Verjüngungen aufweisen (keine Ausführungsformen der Erfindung).

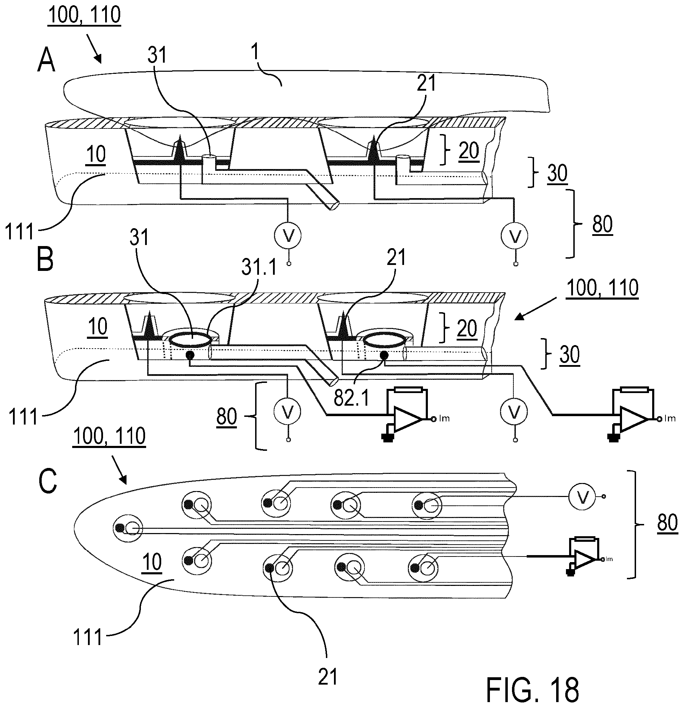

Figur 18 zeigt eine Variante des elektrophysiologisches Messgeräts 100, bei dem die Substrateinrichtung 10, die Elektrodeneinrichtung 20 und Teile, insbesondere die Aktoren, der Stelleinrichtung eine flexible Mess-Sonde 110 bilden (keine Ausführungsform der Erfindung). Es ist in Figur 18 nur das distale Ende der Mess-Sonde 110 gezeigt, die im Übrigen einen flexiblen Schaft zur Einführung z. B. durch ein Endoskop in ein Gewebe aufweist und mit mindestens einer Antriebseinheit zur Ansteuerung der Aktoren der Stelleinrichtung ausgestattet ist. Die Mess-Sonde 110 ist mit allen oben beschriebenen Varianten von Stelleinrichtungen realisierbar, wobei hier beispielhaft auf eine Saugeinrichtung 30 Bezug genommen wird. Bei Betätigung der Stelleinrichtung 30 wird die Zellprobe 1 in Gestalt des Gewebes zu den Nanoelektroden 21 gezogen, so dass diese in die Zellprobe 1 eindringen. Die Mess-Sonde 110 ist z. B. aus flexiblen Polymermaterialien und Metallen aufgebaut. Des Weiteren ist Mess-Sonde 110 kann mit allen oben beschriebenen Varianten von Messeinrichtungen 80, insbesondere Messschaltungen, Pulsgeneratoren, Referenzelektroden, Masse-Elektroden und/oder Verstärkern, ausgestattet sein.

Die Mess-Sonde 110 zur Messung von Membranpotentialen und/oder lonenströmen kann für die Erfassung von intrazellulären Membranpotentialen einzelner neuronaler Zellen und/oder die Messung von transmembranären lonenströmen z. B. in Hirngewebe unter in vivo Bedingungen verwendet werden. Die Verwendung derartiger Sonden und ihre Platzierung im Hirngewebe ist in z. B. in [20] beschrieben. Ein Nachteil der herkömmlichen Sonden besteht darin, dass lediglich die Oberflächenpotentiale von Neuronengruppen, nicht jedoch die intrazellulären Potentiale einzelner Zellen oder die Ströme durch Zellmembranen erfasst werden. Mit der Mess-Sonde 110 werden neue Anwendungen elektrophysiologischer Messgeräte und eine Erweiterung des in [20] beschriebenen Verfahrens erzielt.

Gemäß Figur 18A ist die Mess-Sonde 110 mit einer Vielzahl von Messeinheiten (z. B. Messeinheiten für intrazelluläre Messungen von Membranpotentialen oder Strommessungen durch Membranen in der cell-attached Konfiguration) ausgestattet, die im Wesentlichen wie die oben beschriebenen Varianten des elektrophysiologischen Messgeräts, insbesondere wie in den Figuren 1, 3, 4, 8 und/oder 9, aufgebaut sind (keine Ausführungsformen der Erfindung). In einem Sondenkörper 111 der Mess-Sonde 110, der die Substrateinrichtung 10 bildet, z. B. einen Durchmesser von 1 bis 3 mm aufweist und aus einem flexiblen Polymer hergestellt ist, sind zylinderförmige Vertiefungen eingelassen, die jeweils mit einer Nanoelektrode 21 und einer Saugöffnung 31 ausgestattet sind. Die Nanoelektroden 21 sind über elektrische Leiter mit der Messeinrichtung 80 verbunden. Die Saugöffnungen 31 sind jeweils über eine Saugleitung mit einer Pumpeinrichtung (nicht gezeigt) verbunden. Jede Saugleitung kann mit einem Ventil zur selektiven Ansteuerung einzelner Messeinheiten ausgestattet sein. Die Vertiefungen können für die Messung von intrazellulären Membranpotentialen mit einer ultradünnen Membran (in Figur 18A nicht dargestellt) verschlossen sein (siehe Figur 3). In Bezug auf weitere Einzelheiten der Messeinheiten wird auf die oben beschriebenen Varianten Bezug genommen. Alternativ oder zusätzlich zu den in Figur 18A gezeigten Saugeinrichtungen können andere Stelleinrichtungen vorgesehen sein (siehe z. B. Figuren 8 und 9, keine Ausführungsformen der Erfindung).

Die Mess-Sonde 110 wird durch ein Endoskop oder ein anderes Hohlrohr in das zu untersuchende Gewebe eingeführt. Bei Betätigung der Pumpeinrichtung wird das Gewebe an die Mess-Sonde 110 gezogen, so dass die Nanoelektroden 21 in die Zellen eindringen. Durch das Anlegen eines Unterdrucks wölbt sich das Nervengewebe (oder eine Membran mit dem adhärierten Nervengewebe) in die zylinderförmige Vertiefung, was zur Penetration einzelner Zellen (optional durch die Membran) mit den Nanoelektroden 21 führt.

Um die Ableitung von intrazellulären Potentialen und/oder lonenströmen zu verbessern, können die Substrateinrichtung 10 und die Elektrodeneinrichtung 20 der Mess-Sonde 110 angepasst sein, wie in den Figuren 18B und 18C gezeigt ist (keine Ausführungsform der Erfindung). An den Saugöffnungen 31 in den Vertiefungen des Sondenkörpers ist dieser an seiner Oberfläche mit ringförmigen Aufsätzen 31.1 ausgestattet, die in ihren Abmessungen (Innendurchmesser z.B. 10 µm bis 200 µm) den in den Figuren 4A bis 4D dargestellten Pipettenspitzen entsprechen. Die ringförmigen Aufsätze 31.1 (entsprechend den Pipettenspitzen der oben beschriebenen Ausführungsformen) sind z. B. aus Glas bzw. SiO₂ oder einem Polymer, wie z. B. PDMS, hergestellt und bilden mit der aufgenommenen Zelle einen hochohmigen Kontakt oder Gigaseal-Kontakt. Die ringförmigen Aufsätze 31.1 befinden sich in den zylinderförmigen Vertiefungen, die durch dünne Schläuche (Kapillaren) mit einer oder mehreren Pumpeinrichtungen verbunden sind. Bei der Variante gemäß Figur 18A können auch ringförmige Aufsätze 31.1 (nicht dargestellt) vorgesehen sein, welche die Ränder der Vertiefungen auf der Oberfläche des Sondenkörpers umgeben (keine Ausführungsform der Erfindung).

In Figur 18B sind Merkmale einer weiteren Variante der Mess-Sonde 110 dargestellt, bei der die ringförmigen Aufsätze 31.1 im Randbereich mit mindestens einer Nanoelektrode 21 ausgestattet sind (keine Ausführungsform der Erfindung). In diesem Fall wird der Unterdruck innerhalb des ringförmigen Aufsatzes 31.1 angelegt, was zur Herstellung eines Seals mit der Oberfläche des Nervengewebes führt und gleichzeitig die Penetration von Zellen mit den Nanoelektroden bewirkt.

Figur 18C (keine Ausführungsform der Erfindung) zeigt einen größeren Abschnitt der Mess-Sonde 110 in schematischer Draufsicht mit mehreren Messeinheiten zur Messung von Membranpotentialen (obere Reihe) oder lonenströmen mit der cell-attached-Konfiguration und Membranpotentialen mit Hilfe von Nanoelektroden (untere Reihe). Die Leitungen zu der Messeinrichtung 80 werden innerhalb der Mess-Sonde 110 geführt. Die Saugleitungen oder andere Antriebselemente zur Bewegung der Nanoelektroden sind nicht gezeigt.

Die Figur 19 illustriert eine weitere Varianten der Ausführungsform des erfindungsgemäßen elektrophysiologischen Messgeräts 100. Die Figuren 20 bis 21 illustrieren Varianten des elektrophysiologischen Messgeräts 100 (keine Ausführungsformen der Erfindung). Bei diesen Figuren umfasst das Messgerät 100 jeweils insbesondere eine Substrateinrichtung 10, eine Elektrodeneinrichtung 20 mit einer Nanoelektrode 21, eine Saugeinrichtung 30, eine Messeinrichtung 80 mit einer Masseelektrode 82.1 und eine Kammer 90 zur Aufnahme eines Kultivierungsmediums 2 und der Zellprobe 1 in Gestalt eines Zellaggregats in schematischer Schnittansicht.

Gemäß Figur 19 ist anstelle der in Figur 5 gezeigten Ringöffnung 34 am unteren Ende der Kammer 90 in der Kammerwand 91 ein Ringspalt 34A vorgesehen, der einen ringförmigen Saugkanal bildet. Der Ringspalt 34A hat einen größeren Außendurchmesser als der Durchmesser des unteren Endes der Kammer 90. Die Kammerwand 91 erhält dadurch einen Vorsprung 91C zur weiteren Fixierung der Zellprobe 1 (siehe auch Figur 7). Der Ringspalt 34A ist mit einer Pumpeinrichtung (nicht gezeigt) verbunden, mit welcher der Ringspalt 34A mit einem Unterdruck relativ zum Druck in der Kammer 90 beaufschlagt werden kann. In einer abgewandelten Variante kann der Ringspalt 34A durch mehrere Saugöffnungen ersetzt werden, welche in der Kammerwand gleichmäßig verteilt angeordnet sind.

Bei Beaufschlagung des Ringspalts 34A mit dem Unterdruck erfolgt vorteilhafterweise ein seitliches Ansaugen der Zellprobe 1 (radial nach außen und gleichzeitig hin zum Boden der Kammer 90), was aufgrund der symmetrischen Anordnung des Ringspalts 34A eine Kraftwirkung hin zur Nanoelektrode 21 am Boden der Kammer 90 bewirkt. Obwohl die Nanoelektrode mit einem Winkel von 90 Grad relativ zur Saugrichtung im Ringspalt 34A angeordnet ist, erfolgt ein zuverlässiges Eindringen der Nanoelektrode 21 in die Zellprobe 1.

Die Nanoelektrode 21 ist am Boden der Kammer 90 seitlich neben dem Ringspalt 34A und seitlich neben der mittig vorgesehenen zusätzlichen Perfusionsöffnung angeordnet. Eine Zellmembran der Zellprobe wird somit in der unmittelbaren Umgebung der Nanoelektrode 21 durch den Zellträger 10 gestützt. Vorteilhafterweise kann damit eine Stabilisierung der Zellmembran erzielt werden.

Die am Boden der Kammer 90 mittig angeordnete Perfusionsöffnung ist mit einer Spülleitung 95 verbunden, die einen Perfusionskanal zur Perfusion der Zellprobe 1 und/oder eine Verbindung mit einer Referenzelektrode 82 der Messeinrichtung 80 bereitstellt. Die Spülleitung 95 ist z. B. als Mikrofluidikkanal im Zellträger 10 gebildet.

Figur 20 (keine Ausführungsform der Erfindung) zeigt eine Variante des Messgeräts 100, bei der die Kammer 90 abweichend von der Konusform z. B. gemäß Figur 19 an ihrem unteren Ende die Form eines Kugelschalenabschnitts 91D aufweist. Diese Form hat den Vorteil, dass eine unerwünschte Deformation der Zellprobe 1 z. B. durch einen Vorsprung 91C (siehe Figur 19) oder durch Innenkanten am Boden der Kammer 90 vermieden werden, so dass insbesondere an empfindlichen Zellproben Verletzungen vermieden werden. Der Kugelschalenabschnitt 91D ist vorzugsweise so dimensioniert, dass etwa ein Drittel bis eine Hälfte der angesaugten Zellprobe 1 in dem Kugelschalenabschnitt 91D sitzt. Zellproben 1 in Gestalt von Zellaggregaten haben z. B. einen Durchmesser im Bereich von 300 µm bis 1 mm, so dass der Durchmesser D der Kammer 90 am oberen Ende des Kugelschalenabschnitts 91D und die Höhe des Kugelschalenabschnitts 91D in Axialrichtung vorzugsweise im Bereich 500 µm bis 800 µm gewählt ist.

Zur Bewegung der Zellprobe 1 zur Nanoelektrode 21 am Boden der Kammer 90 ist eine Ringöffnung 34 vorgesehen, die über einen Durchflusskanal 35 unterhalb der Elektrodeneinrichtung 20 mit einer Pumpeinrichtung (nicht gezeigt) verbunden ist. Bei Beaufschlagung mit einem Unterdruck dient die Ringöffnung 34 dem Ansaugen der Zellprobe 1 und deren Fixierung in der Kammer 90.

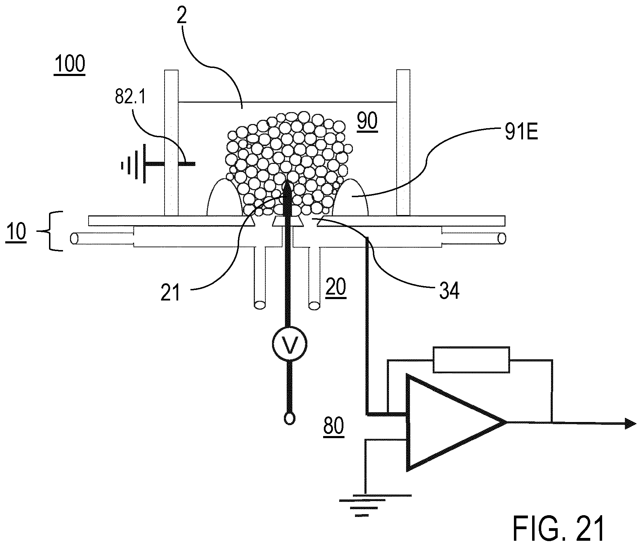

Gemäß Figur 21 (keine Ausführungsform der Erfindung) kann eine zylinderförmige Kammer 90 vorgesehen sein, in deren Boden die Ringöffnung 34 zum Ansaugen der Zellprobe 1 angeordnet ist. Des Weiteren ist am Boden der Kammer 91 eine Ringwulst 91D vorgesehen, die in axialer Richtung nach oben vorsteht und die Ringöffnung seitlich umgibt. Vorteilhafterweise bildet die Ringwulst 91D eine Positionier- und Ansaughilfe bei der Fixierung der Zellprobe 1.

## Patentansprüche

1. Elektrophysiologisches Messgerät (100), das zur intrazellulären Erfassung mindestens eines elektrischen Messwerts an einer biologischen Zellprobe (1) eingerichtet ist, umfassend
- eine Substrateinrichtung (10), die zur Aufnahme der Zellprobe (1) eingerichtet ist,
- eine Elektrodeneinrichtung (20) mit mindestens einer Nanoelektrode (21), die sich jeweils in einer Longitudinalrichtung erstreckt und intrazellulär in die Zellprobe (1) einführbar ist, die von der Substrateinrichtung (10) aufgenommen ist, und
- eine Stelleinrichtung, die für eine Bewegung der mindestens einen Nanoelektrode (21) und/oder der Zellprobe (1) relativ zueinander und entlang der Longitudinalrichtung der mindestens einen Nanoelektrode (21) derart ausgelegt ist, dass die mindestens eine Nanoelektrode (21) in die Zellprobe (1) eindringt, wobei
- die Stelleinrichtung eine Saugeinrichtung (30) umfasst, die für ein Ziehen der Zellprobe (1) zu der mindestens einen Nanoelektrode (21) eingerichtet ist,
- die Saugeinrichtung (30) mindestens eine Saugöffnung (31, 34) in der Substrateinrichtung (10) und eine Pumpeinrichtung (32) umfasst, die zur Beaufschlagung der mindestens einen Saug-öffnung (31, 34) mit einem Unterdruck eingerichtet ist, und
- die mindestens eine Saugöffnung (31, 34) so geformt ist, dass bei Beaufschlagung der mindestens einen Saugöffnung (31, 34) mit dem Unterdruck die Zellprobe (1) entlang der Longitudinalrichtung zu der mindestens einen Nanoelektrode (21) bewegt wird
**dadurch gekennzeichnet, dass**
- oberhalb der Substrateinrichtung (10) eine Kammer (90) mit einer Kammerwand (91) mit einer sich zum Substrat (11) hin verengenden Konusform vorgesehen ist, wobei die Kammer (90) zur Aufnahme eines Kultivierungsmediums (2) und der Zellprobe (1) in Gestalt eines Zellaggregats eingerichtet ist, und
- oberhalb der Substrateinrichtung (10) in der Kammerwand (91) am unteren Ende der Kammer (90) ein Vorsprung (91C) zur Fixierung der Zellprobe (1) vorgesehen ist.

2. Elektrophysiologisches Messgerät gemäß Anspruch 1, bei dem
- die mindestens eine Saugöffnung (31, 34) jeweils mit einem Adhäsionsabschnitt (28) ausgestattet ist, der zur Bildung eines hochohmigen Kontakts oder eines Gigaseal-Kontakt mit einer aufgenommenen Zellprobe (1) eingerichtet ist.

3. Elektrophysiologisches Messgerät gemäß einem der Ansprüche 1 bis 2, bei dem
- die mindestens eine Nanoelektrode (21) auf der Substrateinrichtung (10) vorstehend angeordnet ist, und
- die mindestens eine Saugöffnung (31, 34) zu der mindestens einen Nanoelektrode (21) unmittelbar benachbart angeordnet ist.

4. Elektrophysiologisches Messgerät gemäß Anspruch 3, bei dem
- die Elektrodeneinrichtung (20) eine Vielzahl von Nanoelektroden (21) umfasst, und
- die mindestens eine Saugöffnung (31) zwischen den Nanoelektroden (21) angeordnet ist.

5. Elektrophysiologisches Messgerät gemäß einem der vorhergehenden Ansprüche, bei dem
- die mindestens eine Saugöffnung eine Ringöffnung (34) umfasst, die in der Substrateinrichtung (10) angeordnet ist und entlang einer lateralen Ausdehnung der Substrateinrichtung (10) die mindestens eine Nanoelektrode (21) umgibt, wobei die Ringöffnung (34) für ein Ansaugen der Zellprobe (1) und deren Fixierung an der Substrateinrichtung (10) eingerichtet ist.

6. Elektrophysiologisches Messgerät gemäß einem der vorhergehenden Ansprüche, bei dem
- die mindestens eine Nanoelektrode (21) in der mindestens einen Saugöffnung (31) in der Substrateinrichtung (10) angeordnet ist.

7. Elektrophysiologisches Messgerät gemäß Anspruch 6, bei dem
- die mindestens eine Nanoelektrode (21) sich parallel zu einer Innenwand der Saugöffnung (31, 33, 34) in der Substrateinrichtung (10) erstreckt.

8. Elektrophysiologisches Messgerät gemäß einem der Ansprüche 6 bis 7, bei dem
- die mindestens eine Nanoelektrode (21) so angeordnet ist, dass ihr freies Ende einen vorbestimmten Abstand zur Oberfläche der Substrateinrichtung (10) aufweist.

9. Elektrophysiologisches Messverfahren zur intrazellulären Erfassung mindestens eines elektrischen Messwerts an einer biologischen Zellprobe (1), umfassend die Schritte
- Aufnahme der Zellprobe (1) auf einer Substrateinrichtung (10),
- Anordnung von mindestens einer Nanoelektrode (21) einer Elektrodeneinrichtung (20) derart, dass sie intrazellulär in die Zellprobe (1) ragt, die von der Substrateinrichtung (10) aufgenommen ist, und
- Messung des mindestens einen elektrischen Messwerts an der Zellprobe (1), wobei
- eine Bewegung der mindestens einen Nanoelektrode (21) und/oder der Zellprobe (1) relativ zueinander und entlang der Longitudinalrichtung der Nanoelektroden (21) mit einer Stelleinrichtung (30-70) derart vorgesehen ist, dass die mindestens eine Nanoelektrode (21) in die Zellprobe (1) eindringt, wobei
- die Stelleinrichtung eine Saugeinrichtung (30) umfasst, die für ein Ziehen der Zellprobe (1) zu den Nanoelektroden (21) eingerichtet ist, wobei die Saugeinrichtung (30) mindestens eine Saugöffnung (31, 33, 34) in der Substrateinrichtung (10) und eine Pumpeinrichtung umfasst, die zur Beaufschlagung der mindestens einen Saugöffnung (31, 33, 34) mit einem Unterdruck eingerichtet ist, und
- bei Beaufschlagung der mindestens einen Saugöffnung (31, 33, 34) mit dem Unterdruck die Zellprobe (1) entlang der Longitudinalrichtung zu der mindestens einen Nanoelektrode (21) bewegt wird,
**dadurch gekennzeichnet, dass**
- oberhalb der Substrateinrichtung (10) eine Kammer (90) mit einer Kammerwand (91) mit einer sich zum Substrat (11) hin verengenden Konusform vorgesehen ist, wobei ein Kultivierungsmedium (2) und die Zellprobe (1) in Gestalt eines Zellaggregats in der Kammer (90) aufgenommen wird, wobei
- oberhalb der Substrateinrichtung (10) in der Kammerwand (91) am unteren Ende der Kammer (90) ein Vorsprung (91C) vorgesehen ist, mit dem die Zellprobe (1) fixiert wird.

## Claims

1. Electrophysiological measuring apparatus (100) which is configured for intracellular detection of at least one electrical measuring value on a biological cell sample (1), comprising
- a substrate device (10) configured to receive the cell sample (1),
- an electrode device (20) with at least one nanoelectrode (21) which extends in a longitudinal direction in each case and can be introduced intracellularly into the cell sample (1) which is received by the substrate device (10), and
- an actuating device which is configured for moving of the at least one nanoelectrode (21) and/or the cell sample (1) relative to one another and along the longitudinal direction of the at least one nanoelectrode (21) such that the at least one nanoelectrode (21) penetrates into the cell sample (1), wherein
- the actuating device comprises a suction device (30) configured to draw the cell sample (1) towards the at least one nanoelectrode (21),
- the suction device (30) comprises at least one suction opening (31, 34) in the substrate device (10) and a pump device (32) which is configured to apply a negative pressure to the at least one suction opening (31, 34), and
- the at least one suction opening (31, 34) is shaped such that when the at least one suction opening (31, 34) is subjected to the negative pressure, the cell sample (1) is moved along the longitudinal direction to the at least one nanoelectrode (21),
**characterized in that**
- a chamber (90) with a chamber wall (91) having a conical shape which narrows towards the substrate (11) is provided above the substrate device (10), wherein the chamber (90) is configured to receive a cultivation medium (2) and the cell sample (1) in the form of a cell aggregate, and
- a projection (91C) for fixing the cell sample (1) is provided above the substrate device (10) in the chamber wall (91) at the lower end of the chamber (90).

2. Electrophysiological measuring apparatus according to claim 1, wherein
- the at least one suction opening (31, 34) is each provided with an adhesion portion (28) configured to form a high-impedance contact or a gigaseal contact with a received cell sample (1).

3. Electrophysiological measuring apparatus according to any one of claims 1 to 2, wherein
- the at least one nanoelectrode (21) is arranged protruding on the substrate device (10), and
- the at least one suction opening (31, 34) is arranged immediately adjacent to the at least one nanoelectrode (21).

4. Electrophysiological measuring apparatus according to claim 3, wherein
- the electrode device (20) comprises a plurality of nanoelectrodes (21), and
- the at least one suction opening (31) is arranged between the nanoelectrodes (21).

5. Electrophysiological measuring apparatus according to one of the preceding claims, wherein
- the at least one suction opening comprises a ring opening (34) which is arranged in the substrate device (10) and surrounds the at least one nanoelectrode (21) along a lateral extension of the substrate device (10), wherein the ring opening (34) is configured for suctioning the cell sample (1) and fixing it to the substrate device (10).

6. Electrophysiological measuring apparatus according to one of the preceding claims, wherein
- the at least one nanoelectrode (21) is arranged in the at least one suction opening (31) in the substrate device (10).

7. Electrophysiological measuring apparatus according to claim 6, wherein
- the at least one nanoelectrode (21) extends parallel to an inner wall of the suction opening (31, 33, 34) in the substrate device (10).

8. Electrophysiological measuring apparatus according to one of claims 6 to 7, wherein
- the at least one nanoelectrode (21) is arranged such that its free end comprises a predetermined distance from the surface of the substrate device (10).

9. Electrophysiological measuring method for intracellular detection of at least one electrical measuring value on a biological cell sample (1), comprising the steps of
- receiving the cell sample (1) on a substrate device (10),
- arranging at least one nanoelectrode (21) of an electrode device (20) such that it projects intracellularly into the cell sample (1) which is received by the substrate device (10), and
- measuring the at least one electrical measuring value on the cell sample (1), wherein
- a movement of the at least one nanoelectrode (21) and/or the cell sample (1) relative to one another and along the longitudinal direction of the nanoelectrodes (21) is provided with an actuating device (30-70) such that the at least one nanoelectrode (21) penetrates into the cell sample (1), wherein
- the actuating device comprises a suction device (30) which is configured for drawing the cell sample (1) to the nanoelectrodes (21), wherein the suction device (30) comprises at least one suction opening (31, 33, 34) in the substrate device (10) and a pumping device which is configured for applying a negative pressure to the at least one suction opening (31, 33, 34), and
- when the at least one suction opening (31, 33, 34) is subjected to the negative pressure, the cell sample (1) is moved along the longitudinal direction to the at least one nanoelectrode (21),
**characterized in that**
- a chamber (90) with a chamber wall (91) having a conical shape which narrows towards the substrate (11) is provided above the substrate device (10), wherein a cultivation medium (2) and the cell sample (1) in the form of a cell aggregate are accommodated in the chamber (90), wherein
- a projection (91C) is provided above the substrate device (10) in the chamber wall (91) at the lower end of the chamber (90), with which projection the cell sample (1) is fixed.

## Revendications

1. Appareil de mesure électrophysiologique (100), qui est conçu pour l'acquisition intracellulaire au moins d'une valeur de mesure électrique sur un échantillon de cellules (1) biologique, comprenant
- un dispositif formant substrat (10), qui est conçu pour la réception de l'échantillon de cellules (1),
- un dispositif d'électrode (20) avec au moins une nanoélectrode (21), qui s'étend respectivement dans une direction longitudinale et peut être introduit de manière intracellulaire dans l'échantillon de cellules (1), qui est reçu par le dispositif formant substrat (10), et
- un dispositif de commande, qui est configuré pour un mouvement de l'au moins une nanoélectrode (21) et/ou de l'échantillon de cellules (1) l'une par rapport à l'autre et le long de la direction longitudinale de l'au moins une nanoélectrode (21), de telle sorte que l'au moins une nanoélectrode (21) pénètre dans l'échantillon de cellules (1), dans lequel
- le dispositif de commande comprend un dispositif d'aspiration (30), qui est conçu pour une traction de l'échantillon de cellules (1) vers l'au moins une nanoélectrode (21),
- le dispositif d'aspiration (30) comprend au moins une ouverture d'aspiration (31, 34) dans le dispositif formant substrat (10) et un dispositif de pompage (32), qui est conçu pour la sollicitation de l'au moins une ouverture d'aspiration (31, 34) avec un vide, et
- l'au moins une ouverture d'aspiration (31, 34) est formée de sorte que lors de la sollicitation de l'au moins une ouverture d'aspiration (31, 34) avec le vide l'échantillon de cellules (1) est déplacé le long de la direction longitudinale vers l'au moins une nanoélectrode (21)
**caractérisé en ce que**
- une chambre (90) avec une paroi de chambre (91) avec une forme conique se rétrécissant en direction du substrat (11) est prévue au-dessus du dispositif formant substrat (10), dans lequel la chambre (90) est conçue pour la réception d'un milieu de culture (2) et de l'échantillon de cellules (1) sous forme d'un agrégat cellulaire, et
- une saillie (91C) pour la fixation de l'échantillon de cellules (1) est prévue au-dessus du dispositif formant substrat (10) dans la paroi de chambre (91) sur l'extrémité inférieure de la chambre (90).

2. Appareil de mesure électrophysiologique selon la revendication 1, pour lequel
- l'au moins une ouverture d'aspiration (31, 34) est équipée respectivement d'une section d'adhésion (28), qui est conçue pour la formation d'un contact à haute impédance ou d'un contact gigaseal avec un échantillon de cellules (1) reçu.

3. Appareil de mesure électrophysiologique selon l'une quelconque des revendications 1 à 2, pour lequel
- l'au moins une nanoélectrode (21) est disposée en saillie sur le dispositif formant substrat (10), et
- l'au moins une ouverture d'aspiration (31, 34) est disposée directement au voisinage de l'au moins une nanoélectrode (21).

4. Appareil de mesure électrophysiologique selon la revendication 3, pour lequel
- le dispositif d'électrode (20) comprend une pluralité de nanoélectrodes (21), et
- l'au moins une ouverture d'aspiration (31) est disposée entre les nanoélectrodes (21).

5. Appareil de mesure électrophysiologique selon l'une quelconque des revendications précédentes, pour lequel
- l'au moins une ouverture d'aspiration comprend une ouverture annulaire (34), qui est disposée dans le dispositif formant substrat (10) et entoure l'au moins une nanoélectrode (21) le long d'une étendue latérale du dispositif formant substrat (10), dans lequel l'ouverture annulaire (34) est conçue pour une aspiration de l'échantillon de cellules (1) et la fixation de celui-ci sur le dispositif formant substrat (10) .

6. Appareil de mesure électrophysiologique selon l'une quelconque des revendications précédentes, pour lequel
- l'au moins une nanoélectrode (21) est disposée dans l'au moins une ouverture d'aspiration (31) dans le dispositif formant substrat (10).

7. Appareil de mesure électrophysiologique selon la revendication 6, pour lequel
- l'au moins une nanoélectrode (21) s'étend parallèlement à une paroi intérieure de l'ouverture d'aspiration (31, 33, 34) dans le dispositif formant substrat (10).

8. Appareil de mesure électrophysiologique selon l'une quelconque des revendications 6 à 7, pour lequel
- l'au moins une nanoélectrode (21) est disposée de sorte que son extrémité libre présente une distance prédéfinie par rapport à la surface du dispositif formant substrat (10).

9. Procédé de mesure électrophysiologique pour l'acquisition intracellulaire au moins d'une valeur de mesure électrique sur un échantillon de cellules (1) biologique, comprenant les étapes
- de réception de l'échantillon de cellules (1) sur un dispositif formant substrat (10),
- de disposition d'au moins une nanoélectrode (21) d'un dispositif d'électrode (20), de telle sorte qu'elle fait saillie de manière intracellulaire dans l'échantillon de cellules (1), qui est reçu par le dispositif formant substrat (10), et
- de mesure de l'au moins une valeur de mesure électrique sur l'échantillon de cellules (1), dans lequel
- un mouvement de l'au moins une nanoélectrode (21) et/ou de l'échantillon de cellules (1) l'une par rapport à l'autre et le long de la direction longitudinale des nanoélectrodes (21) avec un dispositif de commande (30-70) est prévu de telle sorte que l'au moins une nanoélectrode (21) pénètre dans l'échantillon de cellules (1), dans lequel
- le dispositif de commande comprend un dispositif d'aspiration (30), qui est conçu pour une traction de l'échantillon de cellules (1) vers les nanoélectrodes (21), dans lequel le dispositif d'aspiration (30) comprend au moins une ouverture d'aspiration (31, 33, 34) dans le dispositif formant substrat (10) et un dispositif de pompage, qui est conçu pour la sollicitation de l'au moins une ouverture d'aspiration (31, 33, 34) avec un vide, et
- lors de la sollicitation de l'au moins une ouverture d'aspiration (31, 33, 34) avec le vide l'échantillon de cellules (1) est déplacé le long de la direction longitudinale vers l'au moins une nanoélectrode (21),
**caractérisé en ce que**
- une chambre (90) avec une paroi de chambre (91) avec une forme conique se rétrécissant en direction du substrat (11) est prévue au-dessus du dispositif formant substrat (10), dans lequel un milieu de culture (2) et l'échantillon de cellules (1) est reçu sous forme d'un agrégat cellulaire dans la chambre (90), dans lequel
- une saillie (91C), avec laquelle l'échantillon de cellules (1) est fixé, est prévue au-dessus du dispositif formant substrat (10) dans la paroi de chambre (91) sur l'extrémité inférieure de la chambre (90).
